# EUROPEAN PATENT SPECIFICATION

(11) **EP 4 110 270 B1**
(45) Date of publication and mention of the grant of the patent: **09.04.2025**
(21) Application number: 21711175.6
(22) Date of filing: 26.02.2021
(51) Int. Cl.: A61K 8/19, A61K 8/27, A61K 8/34, A61K 8/42, A61K 8/44, A61K 8/49, A61K 8/64, A61K 8/67, A61K 8/73, A61K 8/81, A61Q 11/00

(54) **ORAL CARE COMPOSITIONS FOR GUM HEALTH**
MUNDPFLEGEZUSAMMENSETZUNGEN FÜR DIE ZAHNFLEISCHGESUNDHEIT
COMPOSITIONS DE SOIN BUCCAL POUR LA SANTÉ DE LA GENCIVE

(30) Priority: 26.02.2020 WO PCT/CN2020/076760
(43) Date of publication of application: 04.01.2023
(73) Proprietor: The Procter & Gamble Company, Cincinnati, OH 45202 (US)
(72) Inventor: BASCOM, Charles Carson, Cincinnati, Ohio 45202 (US); BIESBROCK, Aaron Reed, Cincinnati, Ohio 45202 (US); ISFORT, Robert Joseph, Cincinnati, Ohio 45202 (US); KLUKOWSKA, Malgorzata, Cincinnati, Ohio 45202 (US); SHI, Yunming, Beijing 101312 (CN); STRAND, Ross, 138547 (SG); TASSEFF, Ryan, Cincinnati, Ohio 45202 (US)
(74) Representative: P&G Patent Germany
(86) International application number: PCT/CN2021/078039
(87) International publication number: WO 2021/170064

(56) References cited:
- EP-A1- 1 393 710
- WO-A1-2019/183876
- WO-A1-2019/183886
- WO-A1-2019/183888
- CN-A- 106 619 332
- CN-A- 108 888 770
- CN-A- 108 939 079
- US-A1- 2015 297 500
- DATABASE GNPD [online] MINTEL; 7 February 2014 (2014-02-07), ANONYMOUS: "Lamiophlomis Rotata Xue Yu Whitening Toothpaste with Salt", XP055809281, retrieved from https://www.gnpd.com/sinatra/recordpage/2305848/ Database accession no. 2305848
- DATABASE GNPD [online] MINTEL; 26 November 2019 (2019-11-26), ANONYMOUS: "Active Mouthwash Gel", XP055809294, retrieved from https://www.gnpd.com/sinatra/recordpage/7062823/ Database accession no. 7062823
- DATABASE GNPD [online] MINTEL; 29 January 2001 (2001-01-29), ANONYMOUS: "Toothpaste", XP055809295, retrieved from https://www.gnpd.com/sinatra/recordpage/85453/ Database accession no. 85453
- DATABASE GNPD [online] MINTEL; 30 January 2019 (2019-01-30), ANONYMOUS: "Lemon Flavoured Gum Protection Biological Peptide Toothpaste", XP055809305, retrieved from https://www.gnpd.com/sinatra/recordpage/6304363/ Database accession no. 6304363
- DATABASE GNPD [online] MINTEL; 10 December 2019 (2019-12-10), ANONYMOUS: "Periodontal Vitamin Toothpaste", XP055809797, retrieved from https://www.gnpd.com/sinatra/recordpage/7088753/ Database accession no. 7088753
- DATABASE GNPD [online] MINTEL; 2 January 2007 (2007-01-02), ANONYMOUS: "Gum-Protection Toothpaste", XP055809798, retrieved from https://www.gnpd.com/sinatra/recordpage/636724/ Database accession no. 636724

## Description

### FIELD OF THE INVENTION

The present invention relates to oral care compositions comprising gum health compound and amino acid as defined in claim 1.

### BACKGROUND OF THE INVENTION

The oral epithelial barrier separates the host from the environment and provides the first line of defense against pathogens, exogenous substances and mechanical stress. The oral epithelial barrier includes underlying connective tissue and a stratified keratinized epithelium with a basement membrane, whose cells undergo terminal differentiation resulting in the formation of a mechanically resistant surface. Gingival keratinocytes are connected by various transmembrane proteins, such as tight junctions, adherens junctions, and gap junctions, each of which has a specialized structure and specific functions. Bacteria secrete compounds detrimental to host defenses, endotoxins and exotoxins, free radicals and collagen-destroying enzymes, leukotoxins, bacterial antigens, waste products, and toxic compounds. Disruption of the gingival epithelial barrier, and the subsequent penetration of exogenous pathogens into the host tissues, triggers an inflammatory response, establishing chronic infection. If this elegant and well-adapted defense system is overwhelmed by bacterial virulence and prolonged inflammation, tissue destruction can be mediated by host cells following stimulation with cytokines and bacterial products. The junctional epithelium migrates apically on the root surface and activates collagen destruction, which eventually leads to periodontal pocket formation and gum recession.

Gum recession is the loss of gum tissue, which attaches to the tooth at the cementoenamel junction (CEJ), which is a slightly visible border that distinguishes the crown of the tooth, which is covered by enamel, and the root of the tooth, which is covered by cementum. When gums recede, gaps can form between the gum tissue and the CEJ, thereby exposing more of the cementum of the tooth's root. Bacteria and debris can build up in the formed gap between the tooth's surface and the gum tissue.

Gum recession can expose the tooth's root, lead to increases in cavities or periodontal disease leading to potential tooth loss, and can be viewed as aesthetically unpleasant. While a dental professional can remove accumulated bacteria, plaque, and/or tartar mechanically, the only way to treat severe gum recession is gum grafting surgery, which can be painful, inconvenient, and expensive. Accordingly, there is a need for a non-surgical treatment option for individuals with high risk of developing or currently diagnosed with gum recession.

CN106619332 A discloses an oral care composition which may prevent gingival recesiion and comprising peptide and carrot extract.

### SUMMARY OF THE INVENTION

This summary is provided to introduce a selection of concepts in a simplified form that are further described below in the detailed description. This summary is not intended to identify required or essential features of the claimed subject matter. Nor is this summary intended to be used to limit the scope of the claimed subject matter.

Disclosed herein is a leave-on oral care composition comprising gum health compound and amino acid.

Disclosed herein is a leave-on oral care composition comprising gum health compound and peptide.

Disclosed herein is an oral care composition comprising gum health compound and amino acid, wherein the composition is a leave-on oral care composition, unit-dose oral care composition, an emulsion composition, a dispersion, a dentifrice, tooth gel, subgingival gel, mouth rinse, mousse, foam, mouth spray, lozenge, chewable tablet, chewing gum, tooth whitening strips, floss and floss coatings, breath freshening dissolvable strips, denture care products, denture adhesive products, or combinations thereof.

Also disclosed herein are kits comprising the disclosed compositions and a suitable delivery carrier.

Also disclosed herein are methods of use of the disclosed compositions to improve the health of the gums in an oral cavity.

Also disclosed herein is a method of preventing gingival recession, stopping gingival recession, reversing gingival recession, decrease gum recession, increasing gingival barrier protection, promoting collagen synthesis, promoting extracellular matrix synthesis, increasing gum resilience, increasing epidermal thickness and/or promoting fibrillin synthesis, or combinations thereof in an oral cavity of an animal comprising contacting the disclosed oral care compositions with at least one surface of the oral cavity.

### BRIEF DESCRIPTION OF THE DRAWINGS

FIG. 1 shows the proportion of the general US population with gum recession according to age.
FIG. 2 shows a variety of gum recession measures.
FIG. 3A shows increasing CAL (cor: 0.64, p: 1.7e-11) with respect to age in the gingival aging clinical population.
FIG. 3B shows decreasing recession (cor: -0.63, p: 1.8e-11) with respect to age in the gingival aging clinical population. in the gingival aging clinical population.
FIG. 4 shows a side by side comparison of (left) a tooth prior to treatment with the disclosed compositions and (right) after six months of treatment with a gum health compound and a pentapeptide. The upward growth of the gingival margin at the tooth midline and the inward migration is visible after treatment.

### DETAILED DESCRIPTION OF THE INVENTION

The present invention is directed to oral care compositions comprising gum health compound in combination with amino acid, as specified in claim 1. Additionally, the present invention is directed to a method of preventing gingival recession, increasing gum resilience, stopping gingival recession, reversing gingival recession, promoting collagen synthesis, promoting fibrillin synthesis, or combinations thereof in an oral cavity of an animal comprising contacting the disclosed oral care compositions with at least one surface of the oral cavity.

Aging has a negative physical impact on many tissues including epithelial tissues such as skin and gingiva. Through a cross-study analysis, common molecular features responsible for the loss of function in aging skin and gingiva were identified. Such features have been well studied in skin and have been pharmacologically targeted to improve aging related damage. However, as described herein, the gingiva has not been well studied. Unexpectedly, when the ingredients found to be successful in improving the health of the dermis were applied to the gums, gingival recession was not only stopped, but gingival recession was reversed. Thus, the oral care compositions described herein provided us with a nonobvious approach to reverse several negative effects of gingival aging including recession.

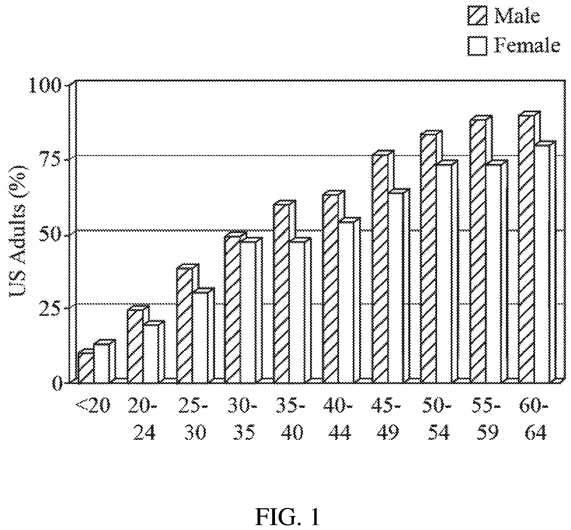

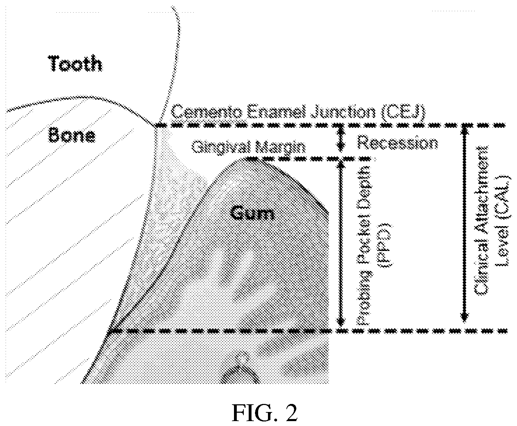

Natural, age-associated gingival recession occurs in nearly all people, as shown in FIG. 1. There are three critical measures for gingival recession, as shown in FIG. 2. Clinical Attachment Level (CAL) is a measurement of gingival recession that depends on the Probing Pocket Depth (PPD). The CAL is a measure of gingival recession that can result from a number of factors including aging and periodontitis. Gingival recession as defined by the relative location of the gingival margin to the Cemento-Enamel Junction (CEJ) is independent of the PPD (Note that the recession metric decreases as the gingival margin recedes below the CEJ such that worsening recession is indicated by a decrease in this metric). This form of gingival recession occurs naturally with age, independent of periodontitis.

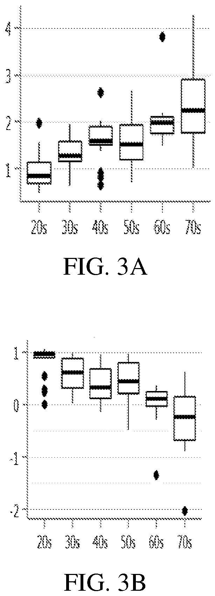

Through natural aging, tissues throughout the human body undergo deleterious physical changes including epithelial tissues such as skin and gingiva. These changes are associated with molecular changes in the expression of important genes and corresponding proteins. To better understand common age-associated molecular changes in two important epithelial tissues, skin and gingiva, we conducted a comparative human clinical study of gene expression change in healthy skin and gingival tissue aging across aged cohorts from 20 years to 70 years in decadal increments. As expected, the studied individuals demonstrated age-associated gingival recession, as shown in FIG. 3.

There are several novel molecular similarities in skin and gingival aging. An analysis of gene expression and a comparison across the aging studies identified similar, statistically significant, decreasing patterns in important collagen genes as a function of age in both skin and gingival tissues. Specifically, we observed age-related decreases in both the major collagen genes, COL1A1 and COL1A2 (shown in Table 1 below).

Collagens are a family of proteins that strengthen and support many tissues in the body, including skin. Type I collagen is the most abundant form of collagen in the human body accounting for 25-35% of all protein in the body, and 75-80% of the protein in skin. The COL1A1 and COL1A2 genes code for proteins that make the type I collagen protein. These proteins cross-link and form the type I collagen fibers that are found in connective tissues throughout the body including skin and the subepithelial connective tissue in gingival (gum) tissue. Importantly, collagen loss in skin is associated with a number of skin aging conditions including laxity, wrinkle formation and dermal thinning. Increasing collagen production is a common target for pharmaceutical agents and cosmetic material compositions for improving skin appearance.

Given the molecular similarities in our comparative aging study, while not wishing to be bound by theory, it was theorized that gingival recession is, at least in part, caused by an ageassociate decrease in collagen expression similar to that which occurs during skin aging. Thus, the loss of collagen in subepithelial connective tissue can result in gingival thinning and loss of gingival fibers, both of which contribute to gingival recession. Furthermore, it is believed that chemical compounds that can increase expression of collagen genes in skin may also reverse age-associated gingival recession.

As such, a human clinical protocol for compound screening tested for the expression of collagen genes after the application of materials to the skin. Several gum health compounds and/or peptides were shown to be effective in promoting collagen expression.

Based on observations from the comparative skin and gingival aging gene expression studies and the theory that collagen loss contributes to both skin and gingival aging, a human clinical study was conducted to evaluate the effect of the combination of retinol and Pal-KTTKS when applied topically in a mucoadhesive gel. Treatment with the oral care compositions disclosed increased the amount of collagen in skin when applied to the gums. Importantly, the combination of a gum health compound, such as retinoid compound, and a peptide demonstrated an unexpectedly high improvement relative to the components applied separately.

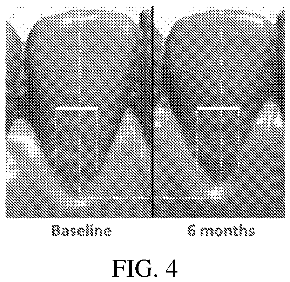

In total, after six months of treatment, the combination of retinol and Pal-KTTKS demonstrated statistically significant improvement in gingival recession by multiple measures, Table 2, when compared to the mucoadhesive vehicle alone, which was visibly observable, as shown in FIG. 4. Surprisingly, increased gingival resilience to damage and/or improved barrier protection was observed as determined by a reduction in post-brushing abrasion.

While some skin care compositions have been disclosed including retinol, skin care compositions are not expected to be suitable for use in the oral cavity due to the inclusion of compounds not suitable for ingestion. Thus, compositions that are dermatologically acceptable, are not necessarily suitable for use as oral care compositions.

Additionally, in many skin care compositions, retinoids can cause some irritation, which would be unacceptable for oral care compositions. Unexpectedly, retinoids were found to improve gum health with minimal or no irritation.

### Definitions

By "oral care composition", as used herein, is meant a product that it is retained in the oral cavity for a time sufficient to contact dental surfaces or oral tissues. Examples of oral care compositions include dentifrice, tooth gel, subgingival gel, mouth rinse, mousse, foam, mouth spray, lozenge, chewable tablet, chewing gum, tooth whitening strips, floss and floss coatings, breath freshening dissolvable strips, denture care products, or denture adhesive products. The oral care composition may also be incorporated onto strips, trays or films for direct application or attachment to oral surfaces.

The term "cone penetration consistency value" as understood herein means the depth, in tenths of a millimeter, that a standard cone will penetrate the sample under fixed conditions of mass, time, and temperature. The cone penetration consistency value is measured according to ASTM method D937-07 first. If the cone penetration consistency value measured according to ASTM method D937-07 is no more than 600, this value is the cone penetration consistency value; if the cone penetration consistency value measured according to ASTM method D937-07 exceeds 600, the cone penetration consistency value is measured according to "Modified version of ASTM method D937-07" specified herein, and this value is the cone penetration consistency value.

The term "delivery carrier" as used herein comprises a material or an appliance that is used to hold the multi-phase oral care composition against the tooth surface. Examples of delivery carriers include strips or dental trays.

The term "strip" as used herein comprises a material 1) whose longest dimension length is generally greater than its width, and 2) whose width is generally greater than its thickness. Strips may be rectangular, arched, curved, semi-circular, have rounded corners, have slits cut into it, have notches cut into it, bent into three dimensional shapes, or combinations thereof. Strips may be solid, semisolid, textured, moldable, flexible, deformable, permanently deformable, or combinations thereof. Strips may be made from plastic sheets including polyethylene, or wax sheets. Examples of strips include a piece of polyethylene about 66mm long, 15mm wide and 0.0178mm thick. Examples of permanently deformable strips include a piece of casting wax sheet about 66mm long, 15mm wide, and 0.4mm thick.

As used herein, the word "or" when used as a connector of two or more elements is meant to include the elements individually and in combination; for example, X or Y, means X or Y or both.

As used herein, the articles "a" and "an" are understood to mean one or more of the material that is claimed or described, for example, "an oral care composition" or "a peptide."

The term "safe and effective amount" as used herein means an amount of a component, high enough to significantly (positively) modify the condition to be treated or to affect the desired whitening result, but low enough to avoid serious side effects (at a reasonable benefit/risk ratio), within the scope of sound medical/dental judgment. The safe and effective amount of a component, will vary with the particular condition being treated, the age and physical condition of the patient being treated, the severity of the condition, the duration of treatment, the nature of concurrent therapy, the specific form employed, and the particular vehicle from which the component is applied.

The term "dispenser", as used herein, means any pump, tube, or container suitable for dispensing oral care compositions.

"Active and other ingredients" useful herein may be categorized or described herein by their cosmetic and/or therapeutic benefit or their postulated mode of action or function. However, it is to be understood that the active and other ingredients useful herein can, in some instances, provide more than one cosmetic and/or therapeutic benefit or function or operate via more than one mode of action. Therefore, classifications herein are made for the sake of convenience and are not intended to limit an ingredient to the particularly stated function(s) or activities listed.

The term "teeth", as used herein, refers to natural teeth as well as artificial teeth or dental prosthesis and is construed to comprise one tooth or multiple teeth. The term "tooth surface" as used herein, refers to natural tooth surface(s) as well as artificial tooth surface(s) or dental prosthesis surface(s) accordingly.

The term "orally acceptable carrier" comprises one or more compatible solid or liquid excipients or diluents which are suitable for use in the oral cavity. By "compatible," as used herein, is meant that the components of the composition are capable of being commingled without interaction in a manner which would substantially reduce the composition's stability and/or efficacy.

While specific reference is made to "consumers" or "patients," throughout the specification, these terms are used interchangeably to refer to any user of the multi-phase oral care composition. The consumer or patient can apply the composition to the oral cavity themselves, or have the composition applied to their oral cavity by a third party, such as a dentist, hygienist, orthodontist, 'or other medical or dental professional.

The term "Gum Care" means those benefits aiming to alleviate one or more symptoms of the earlier stage of gum disease (i.e., gingivitis), which includes: relief of red, swollen, or tender gums; and/or stem gum bleeding.

The term "Gum Health" as used herein refers to inherent or promoted benefits of an oral care composition to provide "Gum Care" benefits that include at least improve gingival and periodontal wound healing, as well as, providing improved gum barrier resilience and strengthening, to mitigate the harmful effects of bacteria as it relates to gum disease, including gingivitis, periodontitis or both.

The term "promoting" as used herein means to promote and/or enhance the Gum Health benefits associated with using the oral care compositions of the present invention in the oral cavity.

The term "substantially free" as used herein refers to the presence of no more than 0.05%, preferably no more than 0.01%, and more preferably no more than 0.001%, of an indicated material in a composition, by total weight of such composition.

The term "essentially free" as used herein means that the indicated material is not deliberately added to the composition, or preferably not present at analytically detectable levels. It is meant to include compositions whereby the indicated material is present only as an impurity of one of the other materials deliberately added.

The term "oral hygiene regimen' or "regimen" can be for the use of two or more separate and distinct treatment steps for oral health. e.g. toothpaste, mouth rinse, floss, toothpicks, spray, water irrigator, massager.

The term "total water content" as used herein means both free water and water that is bound by other ingredients in the oral care composition.

All percentages and ratios used herein after are by weight of total composition (wt%), unless otherwise indicated. All percentages, ratios, and levels of ingredients referred to herein are based on the actual amount of the ingredient, and do not comprise solvents, fillers, or other materials with which the ingredient may be combined as a commercially available product, unless otherwise indicated.

All measurements referred to herein are made at about 23°C (i.e. room temperature) unless otherwise specified.

All parameters that have a method specified herein are measured using the method specified herein, unless otherwise specified.

The oral care compositions, as described herein, comprise gum health compound, peptide, and optionally a mucoadhesive polymer, as claimed. Additionally, the oral care compositions can comprise other optional ingredients, as described below. The section headers below are provided for convenience only. In some cases, a compound can fall within one or more sections. For example, stannous fluoride can be a tin compound and/or a fluoride compound. Additionally, hyaluronic acid, or salts thereof, can be a gum health compound and/or a mucoadhesive polymer.

### Gum Health Compound

The oral care compositions of the present invention comprise gum health compound. A gum health compound is a compound which prevents gingival recession, stops gingival recession, reverses gingival recession, decreases gum recession, increases gingival barrier protection, promotes collagen synthesis, promotes extracellular matrix synthesis, increases gum resilience protection, increases epidermal thickness and/or promotes fibrillin synthesis, or combinations thereof in an oral cavity of an animal comprising contacting gum health compound with at least one surface of the oral cavity.

The gum health compound comprises retinol. Further classes of compounds are discussed in more detail below.

### Vitamin

The oral care composition can comprise one or more vitamins. As used herein, "vitamin" includes all natural and/or synthetic analogs of vitamins, vitamers, compounds and/or derivatives that exhibit the biologically activity of vitamins, isomers of these compounds, stereoisomers of these compounds, salts of these compounds, or combinations thereof.

Suitable vitamins for gum health can include Vitamin A, such as retinoid compound, Vitamin B, including Vitamin B1 (thiamine), Vitamin B2 (riboflavin), Vitamin B3 (niacin), Vitamin B5 (pantothenic acid), Vitamin B6, Vitamin B7 (biotin), Vitamin B9 (folic acid and/or folate), Vitamin B12 (cyanocobalamin), Vitamin C, Vitamin D, Vitamin E, Vitamin K, and/or combinations thereof. Vitamins can also include other vitamin-like compounds, such as choline, carnitine, or combinations thereof.

The oral care composition can comprise from about 0.0001% to about 10%, from about 0.01% to about 5%, or from about 0.01% to about 2%, by weight of the composition, of vitamin.

### Retinoid Compound

The compositions of the present invention can comprise one or more retinoid compounds. As used herein, "retinoid compound" includes all natural and/or synthetic analogs of Vitamin A or retinol-like compounds that possess the biological activity of Vitamin A in the skin as well as the geometric isomers and stereoisomers of these compounds. The retinoid compound can, for example, be retinyl esters (e.g., C-C alkyl esters of retinol, including retinyl palmitate, retinyl acetate, retinyl propionate), retinal, and/or retinoic acid (including all-trans retinoic acid and/or 13-cis-retinoic acid). In some embodiments, retinoids other than retinoic acid are used. These compounds are available in the art and are commercially available from several sources, e.g., Sigma Chemical Company (St. Louis, Mo.), and Boehringer Mannheim (Indianapolis, Ind.). Other suitable retinoids are tocopheryl-retinoate, tocopherol ester of cis- or trans-retinoic acid, adapalene (6-3-(1-adamantyl)- 4-methoxyphenyl-2-naphthoic acid), and tazarotene (ethyl 6-2-(4.4-dimethylthiochroman-6-yl)-ethynylnicotinate). Desirable retinoids include retinol, retinoic acid, retinyl palmitate, retinyl acetate, retinyl propionate, retinal, and combinations thereof.

The retinoid compound may be included as the substantially pure material, or as an extract obtained by suitable physical and/or chemical isolation from natural (e.g., plant) sources. The retinoid compound can be substantially pure, or essentially pure. The compositions of this invention may contain a safe and effective amount of the retinoid compound, such that the oral care composition is safe and effective for regulating or improving the condition of keratinous tissues and accidental ingestion since applied to the oral cavity.

The retinoid compound can comprise retinyl ester, retinal, retinoic acid, tocopheryl-retinoate, tocopherol ester of *cis-* or *trans*-retinoic acid, isotretinoin, alitretinoin, etretinate, acitretin, adapalene, bexarotene, tazarotene, or combinations thereof. The retinoid compound can be pharmaceutical grade, USP, or the like grade, due to use in the oral cavity. The retinoid compound and/or the retinol can have a purity of at least about 95%, at least about 97%, at least about 99%, at least about 99.5%, or at least about 99.9%. The oral care composition can comprise from about 0.0001% to about 10%, from about 0.01% to about 5%, or from about 0.01% to about 2%, by weight of the composition, of retinoid compound. The oral care composition can comprise from about 1 ppm to about 10,000 ppm, from about 500 ppm to about 5000 ppm, from about 750 ppm to about 5000 ppm, from about 1000 ppm to about 2500 ppm, about 1500 ppm, or about 2250 ppm of retinoid compound. Amounts of retinoid compound that are greater than about 5000 ppm are thought to lead to toxicity concerns for formulating with oral care compositions, which would not be present in skin care compositions.

The retinoid compound can comprise retinol comprising *cis-* and/or *trans-* alkene functional groups. The retinol can comprise at least about 80%, at least about 90%, at least about 95%, and/or at least about 99% of *trans*-alkene functional groups.

The retinoid compound can also comprise surfactant, such as anionic surfactant, cationic surfactant, and/or nonionic surfactant, which can improve gum barrier permeability. Suitable surfactants can include polysorbate.

### Metal

The oral care composition of the present invention can comprise a metal. The metal can be provided by a metal ion source. The metal ion source can comprise tin, zinc, copper, or combinations thereof. The metal can provide a gum health benefit. The oral care composition can comprise from about 0.01% to about 5%, from about 0.1% to about 10%, from about 0.001% to about 1%, from about 0.2% to about 1%, from about 0.5% to about 1.5%, or from about 0.3% to about 0.6%, by weight of the oral care composition, of the metal and/or metal ion source.

The metal of the present invention comprise tin, which can be provided by a tin ion source. The tin ion source can be any suitable compound that can provide tin ions in an oral care composition and/or deliver tin ions to the oral cavity when the dentifrice composition is applied to the oral cavity. The tin ion source can comprise one or more tin containing compounds, such as stannous fluoride, stannous chloride, stannous bromide, stannous iodide, stannous oxide, stannous sulfate, stannous sulfide, stannic fluoride, stannic chloride, stannic bromide, stannic iodide, stannic sulfide, and/or mixtures thereof. Preferably, the tin ion source can comprise stannous fluoride, stannous chloride, and/or mixture thereof.

The oral care composition can comprise from about 0.01% to about 5%, from about 0.1% to about 10%, from about 0.001% to about 1%, from about 0.2% to about 1%, from about 0.5% to about 1.5%, or from about 0.3% to about 0.6%, by weight of the oral care composition, of the tin and/or tin ion source.

The metal of the present invention comprise zinc, which can be provided by a zinc ion source. The zinc ion source can comprise one or more zinc containing compounds, such as zinc fluoride, zinc lactate, zinc oxide, zinc phosphate, zinc chloride, zinc acetate, zinc hexafluorozirconate, zinc sulfate, zinc tartrate, zinc gluconate, zinc citrate, zinc malate, zinc glycinate, zinc pyrophosphate, zinc metaphosphate, zinc oxalate, and/or zinc carbonate.

The oral care composition can comprise from about 0.01% to about 5%, from about 0.1% to about 10%, from about 0.001% to about 1%, from about 0.2% to about 1%, from about 0.5% to about 1.5%, or from about 0.3% to about 0.6%, by weight of the oral care composition, of the zinc and/or zinc ion source.

### Gum Strengthening Polyol

The oral care composition of the present invention can comprise a gum-strengthening polyol. The applicant has discovered that gum-strengthening polyols have been shown to additionally strengthen the barrier function of epithelial cells in the oral cavity.

The term "gum-strengthening polyol" can be a sugar alcohol, a disaccharide, a polysaccharide, and preferably a non-reducing sugar. Sugar alcohols are a class of polyols that can be obtained through the hydrogenation of sugar compounds with the formula (CHOH)ₙH₂, where n is seven or more, such as isomalt, maltitol, lactitol, maltotriitol, maltotetraitol, polyglycitol, or combinations thereof. Preferably, n is from about 7 to about 12. Even more preferably, the gum-strengthening polyol is isomalt, maltitol, lactitol, or combinations thereof.

Non-reducing sugars are a class of saccharides that do not generate any compounds containing an aldehyde or ketone functional group. Non-reducing sugars are stable in water and do not react with weak oxidizing agents to produce sugar alcohols. Non-reducing sugars cannot donate electrons to other molecules, and is typically a di, tri, terta, penta saccharide, such as sucrose, trehalose, raffinose, stachyose, verbascose, or combinations thereof. Preferably, the gum-strengthening polyol is trehalose. The gum barrier properties can be obtained from a combination mixture of polyols whereby all have seven or more hydroxyl functional groups.

The oral care composition can comprise from about 0.0001% to about 20%, from about 0.1% to about 18%, from about 0.2% to about 15%, from about 0.5% to about 15%, or from about 1% to about 12%, by weight of the oral care composition, of a gum-strengthening polyol.

The gum-strengthening property can be characterized by Barrier Protection ability as described herein below. For example, a polyol exhibiting the Barrier Protection of more than 10% at concentration of 0.5 wt% can be qualified as a gum-strengthening polyol. In other examples, the gum-strengthening polyol can include polyols having five or more hydroxyl functional groups as long which can provide above-mentioned Barrier Protection ability.

### Allantoin

The oral care composition can comprise from about 0.01% to about 5%, by weight of the composition, of allantoin. Allantoin, also called 5-ureidohydantoin or glyoxyldiureide, is an oxidation product of uric acid. Allantoin helps to heal wounds and skin irritations and stimulates the growth of healthy tissue. In some examples, the allantoin can be present in the amount of from about 0.02% to about 4%, or from about 0.05% to about 3%, by weight of the composition. For example, the allantoin can be present in the amount of about 0.05%, or about 0.1%, or about 0.2%, or about 0.3%, or about 0.4%, or about 0.5%, or about 0.6%, or about 0.8%, or about 0.9%, or about 1%, by weight of the composition. Allantoin can help to improve or increase the wound healing benefit for the hyaluronic acid-containing composition.

### Hydroxy Acid

The oral care compositions may comprise a hydroxy acid. The hydroxy acids can be alphahydroxy or beta-hydroxy acids. Examples of hydroxy acids for use in the compositions of the present invention include salicylic acid, lactic acid, glycolic acid, acetylsalicylic acid, and other salicylic acid derivatives. In some embodiments, salicylic acid and the synthetic and naturally occurring derivatives of salicylic acid can be used in the compositions and methods of the present invention. The hydroxy acid can comprise a compound from Formula I. 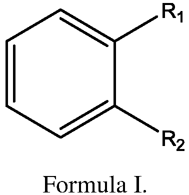 wherein:
R₁ is COOR, or -(CH₂)ₙ-OX;
R is H, or alkyl (e.g., with one to twenty carbons);
n is an integer of about 1 to about 20;
X is H, or 1 to 6 sugar residues (e.g., hexoses or pentoses);
R₂ is COOR, -(CH₂)ₙ-OX, OCO-alkyl (C₁ -C₂₀), or OY; and
Y is H, alkyl (C₁-C₂₀), or 1 to 6 sugar residues (e.g., hexoses or pentoses).

In general, the alkyl groups employed in these hydroxy acid compounds have about one to twenty carbon atoms, although in some embodiments lower alkyl groups are used, for example, alkyl groups with about one to eight carbon atoms. Alkyl groups with even lower numbers of carbon atoms can also be used, for example, alkyl groups with one to six, or one to three carbon atoms.

In some embodiments, salicylic acid is employed in the compositions of the invention. Salicylic acid is a compound of Formula I wherein R1 is COOH and R2 is OH. In other embodiments, acetylsalicylic acid is employed in the compositions of the invention. Acetylsalicylic acid is a compound of Formula I wherein R₁ is COOH and R₂ is OCOCH₃.

When present in the compositions of the present invention, the hydroxy acid can be used in an amount of from about 0.001% to about 50%, or from about 0.01% to about 20%, or from about 0.01% to about 10%, or from about 0.05% to about 5%, or from about 0.05% to about 2% of the composition. According to the invention, *in situ* concentrations of acetylsalicylic acid or salicylic acid that range from about 10⁻⁴ M to about 10⁻⁶ M are effective for increasing cell proliferation and stimulating the production of collagen in mammalian keratinous tissue.

Alternatively, the oral care compositions can be substantially free of, essentially free of, or free of hydroxy acid. The oral care compositions can comprise less than about 0.001% of hydroxy acid.

### Gibberellic Acid

The oral care compositions of the present invention can comprise gibberellic acid. Gibberellic acid comprises a class of compounds that is also referred to as gibberellins. Gibberellins are plant hormones that affect a wide variety of processes throughout the life cycle of plants, including seed germination, stem elongation, flower induction, another development, and seed and pericarp growth. Gibberellins are tetracyclic diterpenoid acids found in fungi and higher plants having the ent-gibberellane ring system.

When present in the compositions of the present invention, gibberellic acids or their derivatives can be used in an amount of from about 0.001% to about 50%, or from about 0.01% to about 20%, or from about 0.01% to about 10%, or from about 0.05% to about 5%, or from about 0.05% to about 2% of the composition. According to the invention, *in situ* concentrations of an active gibberellic acid ranging from about 10⁻⁴ M to about 10⁻⁶ M are effective for increasing cell proliferation and stimulating the production of collagen in mammalian keratinous tissue.

Alternatively, the oral care compositions can be substantially free of, essentially free of, or free of gibberellic acid. The oral care compositions can comprise less than about 0.001% of gibberellic acid.

### Jasmonic Acid

The oral care compositions of the present invention can comprise jasmonic acid compounds. Jasmonic acid compounds employed in the invention include jasmonic acid and jasmonic acid derivatives available to one of skill in the art. Such compounds include jasmonic acid, methyl jasmonate and their isomers. In the present invention jasmonic acid and jasmonic acid derivatives used can include synthetic and natural stereoisomers of jasmonic acid, dihydrojasmonic acid, hydroxy jasmonic acid and dihydro-hydroxy jasmonic acid.

When present in the compositions of the present invention, jasmonic acids or jasmonic acid derivatives can be used in an amount of from about 0.001% to about 50%, or from about 0.01% to about 20%, or from about 0.01% to about 10%, or from about 0.05% to about 5%, or from about 0.05% to about 2% of the composition. According to the invention, in situ concentrations of jasmonic acid ranging from about 10⁻⁴ M to about 10⁻⁶ M are effective for increasing cell proliferation and stimulating the production of collagen in mammalian keratinous tissue.

Alternatively, the oral care compositions can be substantially free of, essentially free of, or free of jasmonic acid compound. The oral care compositions can comprise less than about 0.001% of jasmonic acid compound.

### Zeatin Compounds

The oral care compositions of the present invention can comprise zeatin compounds. Zeatin compounds employed in the invention include the cis and trans isomers of zeatin and the cis and trans isomers of zeatin derivatives available to one of skill in the art. Such zeatin compounds and zeatin derivatives can be natural and synthetic derivatives of the compounds provided by Formula II below. 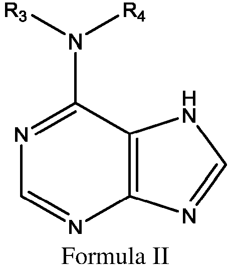
wherein R₃ is H, 3-hydroxymethyl-3-methylallyl, alkyl, -(CH₂)ₙ-CH₃, or OZ;
Z is H, 1 to 6 sugar residues (e.g., hexoses or pentoses), or -(CH₂)ₙ-furan;
R₄ is H, 3-hydroxymethyl-3-methylallyl, alkyl, -(CH)n-C, or OZ; and
n is an integer of from about 1 to about 20.

In some embodiments, zeatin is employed in the compositions of the invention. Zeatin can be a compound of Formula II wherein R₃ is 3-hydroxymethyl-3-methylallyl, and R₄ is H.

When present in the compositions of the present invention, zeatin or its derivatives can be used in an amount of from about 0.001% to about 50%, or from about 0.01% to about 20%, or from about 0.01% to about 10%, or from about 0.05% to about 5%, or from about 0.05% to about 2% of the composition. According to the invention, *in situ* concentrations of zeatin ranging from about 10 M⁻⁴ to about 10⁻⁶ M are effective for increasing cell proliferation and stimulating the production of collagen in mammalian keratinous tissue.

Alternatively, the oral care compositions can be substantially free of, essentially free of, or free of zeatin compound. The oral care compositions can comprise less than about 0.001% of zeatin compound.

### Amino Acid

The oral care composition comprises amino acid as claimed, ie pal-KTTKS. The amino acid can comprise one or more amino acids, peptide, and/or polypeptide, as described herein. Unexpectedly, it has been found that the claimed combination of retinoid compound and amino acid can improve the gum health of a user.

Amino acids, as in Formula III, are organic compounds that contain an amine functional group, a carboxyl functional group, and a side chain (R in Formula III) specific to each amino acid. Suitable amino acids include, for example, amino acids with a positive or negative side chain, amino acids with an acidic or basic side chain, amino acids with polar uncharged side chains, amino acids with hydrophobic side chains, and/or combinations thereof. Suitable amino acids also include, for example, arginine, histidine, lysine, aspartic acid, glutamic acid, serine, threonine, asparagine, glutamine, cysteine, selenocysteine, glycine, proline, alanine, valine, isoleucine, leucine, methionine, phenylalanine, tyrosine, tryptophan, citrulline, ornithine, creatine, diaminobutonic acid, diaminoproprionic acid, salts thereof, and/or combinations thereof.

Suitable amino acids include the compounds described by Formula III, either naturally occurring or synthetically derived. The amino acid can be zwitterionic, neutral, positively charged, or negatively charged based on the R group and the environment. The charge of the amino acid would be well known to one of ordinary skill in the art.

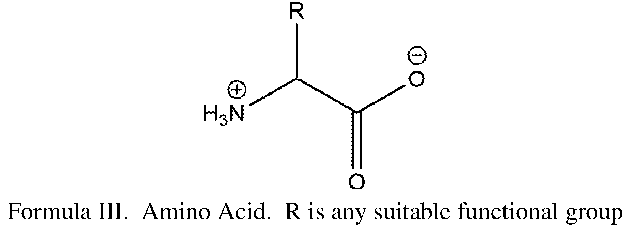

Suitable amino acids include one or more basic amino acids, one or more acidic amino acids, one or more neutral amino acids, or combinations thereof.

The oral care composition can comprise from about 0.01% to about 20%, from about 0.1% to about 10%, from about 0.5% to about 6%, or from about 1% to about 10 % of amino acid, by weight of the oral care composition.

The term "neutral amino acids" as used herein include not only naturally occurring neutral amino acids, such as alanine, asparagine, cysteine, glutamine, glycine, isoleucine, leucine, methionine, phenylalanine, proline, serine, threonine, tryptophan, tyrosine, valine, but also biologically acceptable amino acid which has an isoelectric point in range of pH 5.0 to 7.0. The biologically preferred acceptable neutral amino acid has a single amino group and carboxyl group in the molecule or a functional derivative hereof, such as functional derivatives having an altered side chain albeit similar or substantially similar physio chemical properties. In a further embodiment the amino acid would be at minimum partially water soluble and provide a pH of less than 7 in an aqueous solution of 1g/1000ml at 25°C.

Accordingly, neutral amino acids suitable for use in the invention include, but are not limited to, alanine, aminobutyrate, asparagine, cysteine, cystine, glutamine, glycine, hydroxyproline, isoleucine, leucine, methionine, phenylalanine, proline, serine, taurine, threonine, tryptophan, tyrosine, valine, salts thereof, or mixtures thereof. Preferably, neutral amino acids used in the composition of the present invention may include asparagine, glutamine, glycine, salts thereof, or mixtures thereof. The neutral amino acids may have an isoelectric point of 5.0, or 5.1, or 5.2, or 5.3, or 5.4, or 5.5, or 5.6, or 5.7, or 5.8, or 5.9, or 6.0, or 6.1, or 6.2, or 6.3, or 6.4, or 6.5, or 6.6, or 6.7, or 6.8, or 6.9, or 7.0, in an aqueous solution at 25°C. Preferably, the neutral amino acid is selected from proline, glutamine, or glycine, more preferably in its free form (i.e. uncomplexed). If the neutral amino acid is in its salt form, suitable salts include salts known in the art to be pharmaceutically acceptable salts considered to be physiologically acceptable in the amounts and concentrations provided. Preferably the neutral amino acid is present in the amount of from about 0.0001% to about 10%, preferably from about 0.05% to about 5%, preferably from about 0.1% to about 3%, preferably from about 0.5% to about 3%, preferably from about 1% to about 3%, by weight of the composition. In one aspect, the neutral amino acid is glutamine (or salt thereof). In another aspect, the neutral amino acid is proline (or salt thereof). In yet another aspect, the neutral amino acid is glycine (or salt thereof).

It has been surprisingly discovered that, the use of neutral amino acid increases the collagen synthesis of gingival fibroblasts within a topical leave on application.

The oral care composition can comprise from about 0.0001% to about 20%, from about 0.1% to about 10%, from about 0.5% to about 6%, or from about 1% to about 10 % of neutral amino acid, by weight of the oral care composition.

### Peptide

The oral care composition can also comprise a peptide. A peptide is a linear organic polymer consisting of a number of amino-acid residues bonded together in a chain, forming part of (or the whole of) a protein molecule. The peptide can comprise from two amino acids to ten amino acids, from two amino acids to five amino acids, or from four amino acids to six amino acids.

Peptides, including but not limited to, di-, tri-, tetra-, and pentapeptides and derivatives thereof, may be included in the compositions of the present invention in amounts that are safe and effective, including safe and effective for ingestion. As used herein, "peptides' refers to both the naturally occurring peptides and synthesized peptides. Also, useful herein are naturally occurring and commercially available compositions that contain peptides.

Suitable dipeptides for use herein include, for example, Carnosine (beta-ala-his). Suitable tripeptides for use herein include, for example, gly-his-lys, arg-lys-arg, and/or his-gly-gly. Suitable tripeptide derivatives include palmitoyl-gly-his-lys, which may be purchased as Biopeptide CLTM (100 ppm of palmitoyl-gly his-lys commercially available from Sederma, France); Peptide CK (arg-lys-arg); Peptide CK(ac-arg-lys-arg-NH₂); and a copper derivative of his-gly-gly sold commercially as Iamin, from Sigma (St. Louis, Mo.). Suitable tetrapeptides for use herein include, for example, Peptide E, arg-ser-arg-lys.

Suitable pentapeptides for use herein include lys-thr-thr-lys-ser. A preferred commercially available pentapeptide derivative composition is Matrixyl^{™}, which contains 100 ppm palmitoyl-lys-thr-thr-lys-ser (commercially available from Sederma France).

The peptide can comprise palmitoyl-lys-thr-thr lys-ser, palmitoyl-gly-his-lys, beta-ala-his, their derivatives, and/or combinations thereof. In some embodiments, the peptide comprises palmitoyl-lys-thr-thr-lys-ser, palmitoyl-gly-his-lys, their derivatives, or combinations thereof. In other embodiments, the peptide comprises palmitoyl-lys-thr-thr-lys-ser (pal-KTTKS) and/or derivatives thereof. Other suitable peptides include gly-his-ly (GHK), gly-glu-lys-gly (GEKG), or combinations thereof.

The oral care composition can comprise from about 0.0001% to about 10%, from about 0.01% to about 5%, from about 0.001% to about 5%, from about 0.01% to about 2%, or from about 0.0001% to about 1%, by weight of the composition of peptide. The oral care composition can comprise from about 1 ppm to about 1000 ppm, from about 1 ppm to about 100 ppm, from about 3 ppm to about 50 ppm, or from about 1 ppm to about 10000 ppm, by weight of the oral care composition, of peptide. Amounts of peptide that are greater than about 10000 ppm are thought to lead to toxicity concerns for formulating with oral care compositions, which would not be present in skin care compositions.

### Mucoadhesive Polymer

The oral care composition of the present invention can comprise a mucoadhesive polymer. The mucoadhesive polymer can provide increased retention time on a surface of the oral cavity, such as the gums. Suitable mucoadhesive polymers include polyacrylic acid, natural gum, linear sulfated polysaccharide, chitosan, alginate, anionic cellulose, nonionic cellulose derivative, polyvinyl based homopolymers, poly-vinyl based copolymer or high molecular weight polyethylene oxides (PEO) homopolymers, polypropylene oxide co-polymer, polymers comprising at least a polycarboxylated ethylene backbone, hyaluronic acid or salt thereof and/or combinations thereof.

Polyacrylic acid (PAA) polymer is a generic term for the synthetic high molecular weight polymers of acrylic acid. These may be homopolymers of acrylic acid, crosslinked with an allyl ether pentaerythritol, allyl ether of sucrose or allyl ether of propylene. And, in a water solution at neutral pH, PAA is an anionic polymer, i.e. many of the side chains of PAA will lose their protons and acquire a negative charge. This makes PAAs polyelectrolytes, with the ability to absorb and retain water and swell to many times their original volume. Polyacrylic acid is also called carbomer as tradename. For example, Carbopol^{®}-type polymers, such as Carbopol^{®}, Pemulen^{®} and Noveon^{®}, are polymers of acrylic acid, crosslinked with polyalkenyl ethers or divinyl glycol. Carbomer commercial codes, e.g. 940^{™}, indicate the molecular weight and the specific components of the polymer.

Preferably, a natural gum is selected from the group consisting of gum karaya, gum arabic (also known as acacia gum), gum tragacanth, xanthan gum, alginates, chitosan and combination thereof. More preferably, the natural gum is xanthan gum. Xanthan gum is a polysaccharide secreted by the bacterium *Xanthomonas camestris.* Generally, xanthan gum is composed of a penta-saccharide repeat units, comprising glucose, mannose, and glucuronic acid in a molar ratio of 2:2:1, respectively. The chemical formula (of the monomer) is C₃₅H₄₉O₂₉. In one example, the xanthan gum is from CP Kelco Inc (Okmulgee, US).

Preferably, the hyaluronic acid or a salt thereof is a viscoelastic linear polysaccharide of molecular weight range (1000 to 10,000,000 Da). It is naturally present in the connective tissue of vertebrates, a polymer of glucuronic acid and n-acetyglucosylamine, and is a member of glucosamine family with a high molecular weight. Hyaluronic acid (Hyaluronan) is an indispensable component of intact, healthy gingiva, and oral mucosal tissue. It has many properties that make it a potentially ideal molecule for assisting wound healing by inducing early granulation tissue formation, inhibiting inflammation, promoting epithelial turnover and also connective tissue angiogenesis. Preferably, the hyaluronic acid used in the present invention has a weight average molecular weight (M.W.) of from about 900,000 Daltons to about 5,000,000 Daltons, preferably from about 900,000 Daltons to about 3,000,000 Daltons; more preferably from about 900,000 Daltons to about 2,000,000 Daltons. The molecular weight of the hyaluronic acid can be measured using Gel Electrophoresis method.

Preferably, the linear sulfated polysaccharide is a carrageenan. Examples of carrageenan include Kappa-carrageenan, Iota-carrageenan, Lambda-carrageenan, and combinations thereof. In one example, the linear sulfated polysaccharide is Iota-carrageenan.

Preferably, the anionic cellulose is a carboxymethyl cellulose ("CMC"). In one example, the CMC is prepared from cellulose by treatment with alkali and monochloroacetic acid or its sodium salt. Different varieties are commercially characterized by viscosity. One commercially available example is Aqualon^{™} branded CMC from Ashland Special Ingredients (e.g., Aqualon^{™} 7H3SF; Aqualon^{™} 9M3SF Aqualon^{™} TM9A; Aqualon^{™} TM12A).

Preferably, the nonionic cellulose or derivative thereof has a weight average molecular weight range of about 50,000 Daltons to about 1,300,000 Daltons, and preferably an average degree of polymerization from 300 to 4,800. More preferably, the nonionic cellulose or derivative thereof is hydroxyethyl cellulose ("HEC"). In other examples, the nonionic cellulose may be hydroxypropyl cellulose or hydroxymethyl cellulose.

Preferably the non-ionic synthetic polymers, including but limited to include poly-vinyl based homopolymers, polyvinyl pyrolidone (PVP), polyvinyl acetate (PVA) or poly-vinyl based copolymer e.g. polypyrolidone-co-vinyl acetate (PVP/VA) or high molecular weight polyethylene oxides (PEO) homopolymers (Polyox WSR family) or polypropylene oxide co-polymer (Poloxamers).

Preferably, the polymer comprising at least a polycarboxylated ethylene backbone is selected from the group consisting of co-polymers of maleic anhydride with methyl vinyl ether having a weight average molecular weight of from about 30,000 Daltons to about 1,000,000 Daltons; and co-polymers of maleic acid and acrylic acid or methacrylic.

In an example, the GANTREZ^{™} series of polymers are co-polymers of maleic anhydride with methyl vinyl ether having a weight average molecular weight (M.W.) of about 30,000 Daltons to about 1,000,000 Daltons. These co-polymers are available for example as GANTREZ^{™} AN139 (M.W. 500,000 Daltons), AN119 (M.W. 250,000 Daltons) and S-97 Pharmaceutical Grade (M.W. 70,000 Daltons), from Ashland Chemicals (Kentucky, USA).

In another example, the ACUSOL^{™} and the SOKALAN series of polymers include homopolymers of acrylic acid and copolymers of maleic acid and acrylic acid or methacrylic. Examples are 0:1000 to 1000:0 copolymers of maleic acid with acrylic acid having a weight average molecular weight (M.W.) of about 2,000 to about 1,000,000 Daltons. These copolymers are commercially available as ACUSOL^{™} 445 and 445N, ACUSOL^{™} 531, ACUSOL^{™} 463, ACUSOL^{™} 448, ACUSOL^{™} 460, ACUSOL^{™} 465, ACUSOL^{™} 497, ACUSOL^{™} 490 from Dow Chemicals (Michigan, USA) and as Sokalan^{®} CP 5, Sokalan^{®} CP 7, Sokalan^{®} CP 45, and Sokalan^{®} CP 12 S from BASF (New Jersey, USA).

The use of thiolated mucoadhesive polymer, whereby free thiol groups in the polymer skeleton can help in the formation of the disulfide bonds with that of the cysteine-rich sub-domains present in the mucin which can substantially improve the mucoadhesive properties of the polymers. Thiolated polymers, including but not limited to, chitosan-iminothiolane, poly(acrylicacid)-cystseine, poly(acrylic acid)-homocysteine, chitosan-thioglycolicacid, chitosan-thioethylamidin, alginate-cysteine, sodium carboxymethylcellulose-cysteine.

The oral care composition can comprise from about 0.01% to about 20%, from about 0.01% to about 10%, from about 0.1 to about 8%, or from about 0.001% to about 25%, by weight of the composition of the mucoadhesive polymer.

Additionally or alternatively, the oral care composition can comprise polyacrylic acid an additional polymer, such as natural gum, linear sulfated polysaccharide, anionic cellulose, nonionic cellulose derivative, poly-vinyl based homopolymers, poly-vinyl based copolymer or high molecular weight polyethylene oxides (PEO) homopolymers, polypropylene oxide co-polymer, polymers comprising at least a polycarboxylated ethylene backbone, and/or combinations thereof, in a ratio that provides an optimal viscosity, good adhesion, and retention efficacy that provides a better sensory benefit.

Additionally, the oral care composition can comprise, from about 0.01% to about 5%, from about 0.1% to about 1%, from about 0.01% to about 2%, from about 0.2% to about 0.8%, or from about 0.01% to about 1%, by weight of the composition, of hyaluronic acid, a salt of hyaluronic acid, or combinations thereof.

The oral care composition can comprise from about 0.01% to about 5%, from about 0.5% to about 10%, or from about 0.1 % to about 5%, by weight of the oral care composition, of polyacrylic acid. The oral care composition can comprise from about 0.1% to about 10%, from about 0.5% to about 6%, from about 1% to about 5%, from about 1.3% to about 2.6%, by weight of the composition, of the additional polymer. The additional polymer can comprise natural gum, linear sulfated polysaccharide, anionic cellulose, chitosan, alginate, nonionic cellulose derivative, polyvinyl pyrrolidine, hyaluronic acid or salt thereof, or polymers comprising at least a polycarboxylated ethylene backbone, and combinations thereof.

In some examples, the ratio of the polyacrylic acid and the additional polymer in the oral care composition of the present invention can be from about 5:1 to about 1:5, preferably from about 3:1 to about 1:3. For example, the ratio of the polyacrylic acid and the additional polymer in the present oral care composition, by weight, can be about 4:1, or about 3:1, or about 2:1, or about 1.5:1, or about 1:1, or about 1:1.5, or about 1:2, or about 1:2.5, or about 1:3, or about 1:4, or about 1:5.

The oral care composition described in the present invention is configured for applying on the gingival tissue, as well as other soft tissue (e.g. buccal mucosa) inside the oral cavity of a subject. It has been surprisingly discovered that an oral care composition having both an optimal viscosity and a good adhesion and retention efficacy provides a better sensory benefit to the users, and also is particularly useful for promoting Gum Health benefits to users.

The oral care composition described therein is a leave-on composition. The leave-on composition is applied to the gingival tissue, e.g. gumline area, and left on for more than 2 minutes, preferably more than 10 minutes, more preferably more than 30 minutes and more preferably 60 minutes or longer. Preferably, the leave-on composition is applied to the gingival tissue as the last step of oral hygiene regimen. For example, the leave-on composition of the present invention is applied after brushing teeth, and optionally after using mouth rinse and/or floss.

In one aspect, the present invention is directed to an oral care composition which is in a gel form. It is desirable to have a gel for use in the present invention that enables easy application, thin layer formation and evenly spread into gingival sulcus/pockets and along gingival gum line. The oral care composition has a Viscosity Consistency Coefficient K of about 20 Pa·s to about 500 Pa·s, as measured by the Rheological Test method described herein. Preferably, the oral care composition has a Viscosity Consistency Coefficient K of about 20 Pa·s to about 500 Pa·s, preferably from about 30 Pa·s to about 400 Pa·s, more preferably from about 40 Pa·s to about 300 Pa·s, even more preferably from 50 Pa·s to 250 Pa·s. This optimal viscosity profile range provides better sensory experience of spread ability for a user. If a product is too viscous, it would be hard for a user to spread it evenly onto gingival tissue. If the product has a too low viscosity, it is runny and hard to be retained on appropriate area by finger or applicator. Alternatively, the oral composition can be applied in retained within the use of mouth tray/guard.

In one aspect, the oral care composition of present invention has a desirable mucoadhesion property. Mucoadhesion can be defined as adhesive interaction between two surfaces where one is at least mucosa for a given period through interfacial forces with a consequent decreased in the surface energy. Mucoadhesion polymers for oral care application should ideally (1) easily retain hydrophilic and lipophilic active ingredients and not hinder their release; (2) promote active ingredient penetration and absorption, (3) adhere as quickly as possible to biological substrate and be retained for a period of time, (4) be safe, (5) be cost effective and (6) provide user acceptable application.

The oral care composition of present invention has a Mucoadhesion Index in the range of not less than 0.3 FI%, as measured by the Mucoadhesion Test Method described herein. Preferably, the oral care composition has a Mucoadhesion Index of no less than about 0.5 FI%, and more preferably no less than about 1.0 FI%.

### Fluoride

The oral care composition can comprise fluoride. The fluoride can be provided by a fluoride ion source. The fluoride ion source can comprise one or more fluoride containing compounds, such as stannous fluoride, sodium fluoride, potassium fluoride, amine fluoride, sodium monofluorophosphate, zinc fluoride, and/or mixtures thereof.

The fluoride ion source and the tin ion source can be the same compound, such as for example, stannous fluoride, which can generate tin ions and fluoride ions. Additionally, the fluoride ion source and the tin ion source can be separate compounds, such as when the tin ion source is stannous chloride and the fluoride ion source is sodium monofluorophosphate or sodium fluoride.

The fluoride ion source and the zinc ion source can be the same compound, such as for example, zinc fluoride, which can generate zinc ions and fluoride ions. Additionally, the fluoride ion source and the zinc ion source can be separate compounds, such as when the zinc ion source is zinc phosphate and the fluoride ion source is stannous fluoride.

The oral care composition can comprise a fluoride ion source capable of providing from about 50 ppm to about 3500 ppm, and preferably from about 500 ppm to about 3000 ppm of free fluoride ions. To deliver the desired amount of fluoride ions, the fluoride ion source may be present in the total oral care composition at an amount of from about 0.0025% to about 5%, from about 0.2% to about 1%, from about 0.5% to about 1.5%, or from about 0.3% to about 0.6%, by weight of the oral care composition.

### Other Ingredients

The term "orally acceptable carrier" as used herein means a liquid or semi-solid vehicle such as a paste or a gel for containing the active ingredients of the present invention and delivering them to the oral cavity. Water is commonly used as a carrier material in oral compositions due to its many benefits. For example, water is useful as a processing aid, is benign to the oral cavity and assists in quick foaming of toothpastes. Water may be added as an ingredient in its own or it may be present as a carrier in other common raw materials such as, for example, sorbitol and sodium lauryl sulphate. The term total water content as used herein means the total amount of water present in the oral care composition, whether added separately or as a solvent or carrier for other raw materials but excluding that which may be present as water of crystallization in certain inorganic salts.

The oral care composition of the present invention comprises at least about 30% of a total water content. Preferably, the oral care composition comprises from more than about 35% to about 85% of a total water content. In other embodiments, the compositions include from about 40% to about 80%, alternatively from about 45% to about 75%, alternatively from about 50% to about 70%, alternatively from about 50% to about 60 %, alternatively from about 45% to about 55%, alternatively from about 55% to about 65%, alternatively from about 65% to about 75%, alternatively combinations thereof, of a total water content.

The oral care compositions described herein may contain humectants. The humectants serve to keep the oral care composition from hardening upon exposure to air, to give a moist feel to the mouth, and, for particular humectants, to impart a desirable sweetness of flavor.

Suitable humectants for the present invention include edible polyhydric alcohols such as glycerin, sorbitol, xylitol, erythritol, butylene glycol, polyethylene glycol, propylene glycol, and combinations thereof. In one embodiment, the humectant is selected from sorbitol, glycerin, and combinations thereof. In another embodiment, the humectant is glycerin. In yet another embodiment, the humectant is sorbitol. In one embodiment, the oral care composition comprises from about 1% to less than about 50% of humectants by weight of the composition, preferably from about 10% to about 40%. In yet another embodiment, the oral care composition contains from about 15% to about 30% of glycerin by weight of the oral care composition.

In one example, the oral care composition of the present invention is substantially free of ethanol, preferably essentially free of ethanol. Ethanol is not desirable, in some cases, as it may cause irritating feeling to the users.

Preferably, the oral care composition of the present invention is substantially free of abrasives. The term "abrasive", for the purpose of present invention, includes calcium-containing abrasives and silica abrasives. The calcium-containing abrasives may be selected from the group consisting of calcium carbonate, dicalcium phosphate, tricalcium phosphate, calcium orthophosphate, calcium metaphosphate, calcium polyphosphate, calcium oxyapatite, sodium carbonate, and combinations thereof. In one embodiment where the calcium-containing abrasive is calcium carbonate, the calcium carbonate can be selected from the group consisting of fine ground natural chalk, ground calcium carbonate, precipitated calcium carbonate, and combinations thereof. The silica abrasives may generally have an average particle size ranging from 0.1 to 30 µm, and preferably from 5 to 15 µm. The silica abrasives can be precipitated silica or silica gels such as the silica xerogels marketed under the trade name "Syloid" by the W.R. Grace & Company, Davison Chemical Division and precipitated silica materials such as those marketed by the J.M. Huber Corporation under the trade name, Zeodent^{®}, particularly the silicas carrying the designation Zeodent^{®} 119, Zeodent^{®} 118, Zeodent^{®} 109 and Zeodent^{®} 129.

Preferably, the oral care composition of the present invention contains low levels of abrasives. For example, the oral care composition may comprise from 0% to about 5% by weight of the composition, of abrasives, alternatively from 0% to about 3%, alternatively from 0% to about 2%, alternatively from 0% to about 1%, alternatively less than about 3%, or less than about 2%, or less than about 1%, or less than about 0.5%, by weight of the composition. Preferably, the composition is substantially free of the abrasives, more preferably free of the abrasives.

The oral care compositions herein may include from about 0.001% to about 5%, alternatively from about 0.01% to about 4%, alternatively from about 0.1% to about 3%, alternatively from about 0.5% to about 2%, alternatively combination thereof, of a flavorant composition by weight of the oral care composition. The term flavorant composition is used in the broadest sense to include flavor ingredients, or sensates, or sensate agents, or combinations thereof. Flavor ingredients may include those described in U.S. Publication No. 2012/0082630A1 at paragraph 39; and sensates and sensate ingredients may include those described at paragraphs 40 - 45.

Examples of flavor compositions or flavor ingredients include: mint oils, wintergreen, clove bud oil, cassia, sage, parsley oil, marjoram, lemon, orange, propenyl guaethol, heliotropine, 4-cis-heptenal, diacetyl, methyl-p-tert-butyl phenyl acetate, methyl salicylate, ethyl salicylate, 1-menthyl acetate, oxanone, a-irisone, methyl cinnamate, ethyl cinnamate, butyl cinnamate, ethyl butyrate, ethyl acetate, methyl anthranilate, iso-amyl acetate, iso-amyl butyrate, allyl caproate, eugenol, eucalyptol, thymol, cinnamic alcohol, octanol, octanal, decanol, decanal, phenylethyl alcohol, benzyl alcohol, a-terpineol, linalool, limonene, citral, neral, geranial, geraniol nerol, maltol, ethyl maltol, anethole, dihydroanethole, carvone, menthone, beta -damascenone, ionone, gamma -decalactone, gamma -nonalactone, y-undecalactone, or combinations thereof. Generally suitable flavor ingredients are chemicals with structural features and functional groups that are less prone to redox reactions. These include derivatives of flavor ingredients that are saturated or contain stable aromatic rings or ester groups.

Sensates such as cooling, warming, and tingling agents are useful to deliver signals to the users. The most well-known cooling agent is menthol, particularly 1-menthol, which is found naturally in peppermint oil. Among synthetic cooling agents, many are derivatives of or are structurally related to menthol, i.e., containing the cyclohexane moiety, and derivatized with functional groups including carboxamide, ketal, ester, ether and alcohol. Examples include the p-menthanecarboxamide compounds such as N-ethyl-p-menthan-3-carboxamide (known commercially as "WS-3"). An example of a synthetic carboxamide cooling agent that is structurally unrelated to menthol is N,2,3-trimethyl-2-isopropylbutanamide. Additional exemplary synthetic cooling agents include alcohol derivatives such as 3-1-menthoxy-propane-1,2-diol, isopulegol, p-menthane-3,8-diol; menthone glycerine acetal (known commercially as "MGA"); menthyl esters such as menthyl acetate, menthyl acetoacetate, menthyl lactate, and monomenthyl succinate.

Additional agents that are structurally unrelated to menthol but have been reported to have a similar physiological cooling effect include alpha-keto enamine derivatives described in U.S. Pat. No. 6,592,884, including 3-methyl-2-(1-pyrrolidinyl)-2-cyclopenten-1-one (3-MPC), 5-methyl-2-(1-pyrrolidinyl)-2-cyclopenten-1-one (5-MPC); 2,5-dimethyl-4-(1-pyrrolidinyl)-3(2H)-furanone (DMPF); icilin (also known as AG-3-5, chemical name 142-hydroxyphenyl]-4-[2-nitrophenyl]-1,2,3,6-tetrahydropyrimidine-2-one).

Some examples of warming agents include ethanol; nicotinate esters, such as benzyl nicotinate; polyhydric alcohols; nonanoyl vanillyl amide; nonanoic acid vanillyl ether; vanillyl alcohol alkyl ether derivatives such as vanillyl ethyl ether, vanillyl butyl ether, vanillyl pentyl ether, and vanillyl hexyl ether; isovanillyl alcohol alkyl ethers; ethylvanillyl alcohol alkyl ethers; veratryl alcohol derivatives; substituted benzyl alcohol derivatives; substituted benzyl alcohol alkyl ethers; vanillin propylene glycol acetal; ethylvanillin propylene glycol acetal; ginger extract; ginger oil; gingerol; zingerone; or combinations thereof.

Examples of some tingling agents include capsaicin; homocapsaicin, jambu oleoresin, zanthoxylum peperitum, saanshool-I, saanshool II, sanshoamide, piperine, piperidine, spilanthol, 4-(1-methoxymethyl)-2-phenyl-1,3-dioxolane, or combinations thereof.

The oral care compositions herein can further include herbal ingredients such as extracts of chamomile, oak bark, melissa, rosemary and salvia. These, and some of the herb-derived flavoring components can be included at levels just sufficient to provide a contribution to the flavor or they can be added at higher levels, such as 1% or more, in order to provide a greater therapeutic effect.

The oral care composition of the present invention may comprise preservatives. The preservatives may be benzyl alcohol, phenoxyethanol, sorbitan caprylate (Velsan SC^{®}), 1-2 hexanediol & caprylyl glycol (Symdiol 68^{®}), parabens and or combinations. The paraben may comprise methyl paraben or propyl paraben or combination thereof. Levels of benzyl alcohol or phenoxyethanol may be present at the amount of from greater than about 0.10% to about 0.40%, preferably about 0.15% to about 0.30%, and/or more preferably about 0.15% to about 0.20%. The levels of Velsan C^{®} maybe present at the amount of from about 0.10% to about 0.50%, preferably from about 0.20% to about 0.40%, more preferably alternatively from about 0.25% to about 0.30%. The level of Symdiol 68^{®} maybe present from about 0.10% to about 0.80%, preferably from about 0.10% to about 0.50% and more preferably about 0.20% to about 0.30%. Levels of paraben may be present at the amount of about 0.01% to about 0.20%, preferably about 0.02% to about 0.15%, more preferably about 0.05% to about 0.10%, by weight of the composition.

In one embodiment, the oral care compositions of the present invention are substantially free of triclosan (i.e., 5-chloro-2-(2,4-dichlorophenoxy)phenol), preferably free of triclosan.

The oral care compositions herein may include a sweetening agent. These include sweeteners such as saccharin, dextrose, sucrose, lactose, xylitol, maltose, levulose, aspartame, sodium cyclamate, D-tryptophan, dihydrochalcones, acesulfame, sucralose, neotame, and mixtures thereof. Sweetening agents are generally used in oral care compositions at levels of from about 0.005% to about 5%, alternatively about 0.01% to about 1%, by weight of the composition, alternatively from about 0.1% to about 0.5%, alternatively combinations thereof.

The oral care compositions herein may include a coloring agent *(i.e.,* pigments, dyes and opacifiers). The coloring agent may be in the form of an aqueous solution, preferably 1% coloring agent in a solution of water. Titanium dioxide may also be added to the present oral care composition. Titanium dioxide is a white powder which adds opacity to the oral care compositions. Titanium dioxide generally comprises from about 0.25% to about 5%, by weight of the composition. It will be appreciated that selected components for the compositions must be chemically and physically compatible with one another.

The oral care compositions may include an effective amount of an anti-calculus agent, which in one embodiment may be present from about 0.05% to about 50%, alternatively from about 0.75% to about 25%, alternatively from about 0.1% to about 15%. Non-limiting examples include those described in U.S. Publication No. 2011/0104081A1 at paragraph 64, and those described in U.S. Publication No. 2012/0014883A1 at paragraphs 63 to 68, as well as the references cited therein. One example is a pyrophosphate salt as a source of pyrophosphate ion. In one embodiment, the composition comprises tetrasodium pyrophosphate (TSPP) or disodium pyrophosphate or combinations thereof, preferably from about 0.01% to about 2%, more preferably from about 0.1% to about 1% of the pyrophosphate salt by weight of the composition. Without wishing to be bound by theory, TSPP may provide not only calcium chelating thereby mitigating plaque formation, but also may also provide the additional benefit of monofluorophosphate stabilization (in those formulations containing monofluorophosphate).

The ingredients may provide additional benefits to the oral care composition. The use of extracellular antioxidants, e.g. ascorbate or α-tocopherol, as chain breaking or radical scavenging antioxidants, helps control and modulate the intracellular reactive oxygen species (ROS) that can cause host-tissue damage. The use of dexpanthenol can improve epithelialization and induce the proliferation phase in the wound healing of damaged tissue.

The present oral care composition may further comprise the usual and conventional ancillary components such as surfactants, anti-microbial agents, fluoride ions, and other ingredients that are known to one skilled in the art. It will be appreciated that selected components for the oral care compositions must be chemically and physically compatible with one another.

### Method of Use

The present invention also relates to methods for treating the oral cavity comprising applying to the intraoral tissue (e.g. oral mucosa, gingiva) of the oral cavity of a subject, particularly the gum tissue, leaving on for more than 2 minute, preferably more than 10 minutes, more preferably more than 30 minutes, or more than 60 minutes or longer. The method of use herein comprises contacting a subject's oral mucosa (e.g. gingival margin or gingival sulcus/pockets) with the oral care composition according to the present invention.

The present invention further relates to a method of improving Gum Health of a subject using the oral care composition described herein comprising the step of applying the oral care composition onto the intraoral tissue of a subject, preferably applying along the gingival margin or sulcus at least once a day, preferably at least twice a day, more preferably every time immediately after brushing teeth. The term "immediately" herein means within 1 hour, preferably within 30 minutes, more preferably within 15 minutes, alternatively within 10 minutes.

The oral care composition can be applied onto the intraoral tissue of a subject by using an applicator, which applicator has a handle and a head, to spread the oral care composition along the gingival margin or sulcus of the subject. Preferably, the oral care composition is applied on the head of the applicator before being applied onto the intraoral tissue.

To further increase residence contact time between the oral gingiva and the oral care composition, then the oral composition can first be can be applied into a mouth guard tray, which is then placed into the mouth for upper, lower or both and worn preferably more than 30 minutes, or more than 60 minutes or longer and more preferably overnight.

The present invention further relates to a method of improving Gum Health of a subject, comprising at least two steps: (a) brushing teeth with an antibacterial toothpaste, preferably a stannous containing toothpaste, and immediately followed by (b) applying the oral are composition as defined herein onto the intraoral tissue of a subject, preferably applying along the gingival margin, sulcus or pockets.

The present invention further relates to a method of improving Gum Health of a subject, comprising at least two steps: (a) directing a user to apply a dentifrice composition to an oral cavity, and followed by (b) directing the user to apply an oral care composition comprising gum health composition, as described herein, onto the intraoral tissue of a subject, preferably applying along the gingival margin, sulcus or pockets. The dentifrice composition can comprise a metal ion source, such as a tin, zinc, or combinations thereof, and/or fluoride. The oral care composition can comprise amino acid and/or mucoadhesive polymer. The oral care composition can also be a leave-on oral care composition to increase the contact time to more effectively deliver the gum health compound to the gingival margin.

The direction to apply certain composition can be provided by an oral health professional, such as a dentist or a dental hygienist, and/or provided by directions listed on packaging materials for either composition or a kit including both compositions.

In practicing the present invention, the patient applies the oral care composition, as described herein, that contains the gum health compound to obtain the desired effect, such as, treating gum recession, to the oral cavity. The composition can be applied with a paint-on device, a syringe or unit dose syringe, squeezable tube, a brush, a pen or brush tip applicator, a doe's foot applicator, swab, lip gloss applicator, strip that is removed after application, tray that is removed after application, or the like, or even with the fingers. The composition can also be combined with a delivery carrier, such as a strip of material, a dental tray, or a sponge material, and thereafter applied to the oral cavity. In certain embodiments, the compositions or delivery systems herein are almost unnoticeable when applied to oral cavity. After a desired period of time has elapsed, any residual composition may be easily removed by wiping, brushing or rinsing the oral surface. Alternatively, the residual composition can be left in place contacting the oral surface, indefinitely.

Dental tray appliances may be used as follows. The patient or dental professional dispenses the present composition into a soft or rigid dental appliance and then the participant places the appliance over the participant's dental arch (or fits the device around his or her teeth to keep the tray in position). Generally, the recommended treatment period is a sufficient period of time to achieve the benefit, as described herein. At the end of the treatment period, the dental appliance is removed, cleaned with water to remove any remaining composition, and then stored until the next application.

The described compositions and delivery systems, described herein, may be combined in a kit which comprises: 1. present composition and 2. instructions for use; or which comprises: 1. present composition, 2. instructions for use, and 3. a delivery carrier.

### Delivery Carrier

The present invention may further be related to a delivery system or methods for delivering the oral care composition directly to the oral cavity or to the gingiva in the oral cavity of a consumer. The oral care composition can be used in combination with a re-usable delivery carrier, such as a tray, applicator, mouth guard, retainer, or combinations thereof. As the delivery carrier may be re-usable, it is desirable for the oral care composition to be rinseable or water-dispersible, as described herein. The oral care compositions can also be used in combination with a disposable or single-use delivery carrier, such as a disposable strip.

For example, the delivery system may comprise a first layer of a carrier material and a second layer comprising an oral care composition described herein, whereby the gum health compound and/or amino acid is releasably located within the present composition. A suitable first layer may comprise a delivery carrier including a strip of material, a dental tray, a sponge material, and mixtures thereof. In certain embodiments, the delivery carrier may be a strip of material, such as a permanently deformable strip. Suitable strips of material or permanently deformable strips are for example disclosed in U.S. Pat. Nos; 6,136,297; 6,096,328; 5,894,017; 5,891,453; and 5,879,691; and in U.S. Pat. Nos. 5,989,569 and 6,045,811; and in patent application US 2014/0178443 A1.

The delivery carrier may comprise a dissolvable film, such as the dissolvable film strip disclosed in US 6,709,671, which can be adhered to the oral cavity thereby releasing an active, the dissolvable film comprising water-soluble polymers, one or more polyalcohols, and one or more actives. In addition to one or more actives, a dissolvable film may contain a combination of certain plasticizers or surfactants, colorants, sweetening agents, flavors, flavor enhancers, or other excipients commonly used to modify the taste of formulations intended for application to the oral cavity. The resulting dissolvable film is characterized by an instant wettability which causes the dissolvable film to soften soon after application to the mucosal tissue, thus preventing the patient from experiencing any prolonged adverse feeling in the mouth, and a tensile strength suitable for normal coating, cutting, slitting, and packaging operations.

The dissolvable film may comprise a water-soluble polymer or a combination of water-soluble polymers, one or more plasticizers or surfactants, one or more polyalcohols, and an active. The polymers used for the dissolvable film include polymers which are hydrophilic and/or water-dispersible. Examples of polymers that can be used include polymers that are water-soluble cellulose-derivatives, such as hydroxypropylmethyl cellulose, hydroxyethyl cellulose, or hydroxypropyl cellulose, either alone, or mixtures thereof. Other optional polymers, without limiting the invention, include polyvinyl pyrrolidone, carboxymethyl cellulose, polyvinyl alcohol, sodium alginate, polyethylene glycol, natural gums like xanthan gum, tragacantha, guar gum, acacia gum, arabic gum, water-dispersible polyacrylates like polyacrylic acid, methylmethacrylate copolymer, carboxyvinyl copolymers. The concentration of the water-soluble polymer in the final film can very between 20 and 75% (w/w), or between 50 and 75% (w/w).

The strip of material may contain shallow pockets. When the multi-phase oral care composition is coated on a strip of material, bleach agents and/or oral care actives, fill shallow pockets to provide reservoirs of additional bleach agents and/or oral care actives. Additionally, the shallow pockets help to provide texture to the delivery system. The strip of material may have an array of shallow pockets. Generally, the shallow pockets are approximately 0.4 mm across and about 0.1 mm deep. When shallow pockets are included in the strip of material and multi-phase oral care compositions herein are applied to it in various thicknesses, in certain embodiments the overall thickness of the delivery system is less than about 1 mm, in certain embodiments the overall thickness is less than about 0.5 mm.

The delivery systems as used herein may comprise an adhesion means, such that they are capable of adhesion to oral surfaces, especially the teeth. This adhesion means may be provided by the present compositions herein or the adhesion means may be provided independently of the compositions herein (for example the adhesion means is a separate phase from the compositions herein where the compositions may also have an adhesive means). The strip of material may be held in place on the oral surface by adhesive attachment provided by the present composition. The viscosity and general tackiness of the multi-phase oral care composition to dry surfaces may cause the strip to be adhesively attached to the oral surface without substantial slippage from the frictional forces created by the lips, teeth, tongue, and other oral surfaces rubbing against the strip of material while talking drinking, etc. However, this adhesion to the oral surface may be low enough to allow the strip of material to be easily removed by the wearer by simply peeling off the strip of material using one's finger. The delivery system may be easily removable from the oral surfaces without the use of an instrument, a chemical solvent or agent or excess friction.

In addition, the strip of material may be held in place on the oral surface by adhesive means and attachment provided by the delivery carrier itself. For example, the strip of material can extend, attach, and adhere to the oral soft tissue. In addition, an adhesive can be applied to that portion of the strip of material that will attach the delivery systems to the oral soft tissue. The delivery carrier may also be attached to the oral cavity by physical interference or mechanical inter-locking between the delivery carrier and the oral surfaces including the teeth. In addition, the strip of material may be held in place by an adhesion means that is independent of the composition of the present inventions herein, as disclosed in WO 03/015656.

Suitable adhesion means are known to the skilled person. When the adhesive means, if present, is provided by an adhesive, the adhesive may be any adhesive which may be used to adhere materials to the tooth surface or to a surface of the oral cavity surfaces. Suitable adhesives include, but are not limited to, skin, gum and muco adhesives, and should be able to withstand the moisture, chemicals and enzymes of the oral environment for long enough for the oral care actives and/or bleach to take effect, but may be soluble and/or biodegradable thereafter. Suitable adhesives may for example comprise water soluble polymers, hydrophobic and/or non-water-soluble polymers, pressure and moisture sensitive adhesives, e.g. dry adhesives which become tacky upon contact with the mouth environment, e.g. under the influence of moisture, chemicals or enzymes etc. in the mouth. Suitable adhesives include natural gums, synthetic resins, natural or synthetic rubbers, those gums and polymers listed above under "Thickening Agents", and various other tacky substances of the kind used in known adhesive tapes, those known from US 2,835,628.

In addition, the delivery system may comprise an optional release liner. Such a release liner may be formed from any material which exhibits less affinity for the second layer composition than the second layer composition exhibits for itself and for the first layer strip of material. The release liner may comprise a rigid sheet of material such as polyethylene, paper, polyester, or other material, which is then coated with a nonstick type material. The release liner may be cut to substantially the same size and shape as the strip of material or the release liner may be cut larger than the strip of material to provide a readily accessible means for separating the material from the strip. The release liner may be formed from a brittle material that cracks when the strip is flexed or from multiple pieces of material or a scored piece of material. Alternatively, the release liner may be in two overlapping pieces such as a typical adhesive bandage design. A description of materials suitable as release agents is found in Kirk-Othmer, Encyclopedia of Chemical Technology, Fourth Edition, Volume 21, pp. 207-218.

The delivery carrier may be a permanently deformable strip of material having a yield point and thickness such that the strip of material substantially conforms to a shape of a tooth via permanent deformation under a pressure less than about 250,000 Pascals as it has been found that wearers will press a strip onto each tooth using one fingertip having about one square centimeter surface area. They typically apply force at each tooth for one second or less with a typical application pressure ranging from about 100,000 Pascals to about 250,000 Pascals.

The strip of material can have visco-elastic properties which enable it to creep as well as bend in order to conform across several teeth and around the arch of the wearer's mouth. It is important that the necessary permanent deformation occurs under minimum normal force being applied by the wearer.

The deformable strip of material may be made of a permanently deformable material, such as wax, putty, tin or foil, as a single layer or a combination of layers or materials, such as a laminate. In certain embodiments, the deformable strip may be wax, such as #165 sheet wax formulated and manufactured by Freeman Mfg. & Supply Co. of Cleveland, Ohio. This particular wax readily conforms to the shape of a tooth under a pressure of about 133,000 Pascal which is the pressure generated when the wearer applies a normal force of about 3 pounds (1.36 kg) over an area of about one square centimeter. The deformable strip of material may have a nominal film thickness of about 0.8 mm, wherein the deformable strip may be substantially flat and rectangular in shape with rounded corners. The deformable strip of material may have a length sufficient to cover a plurality of adjacent teeth while conforming to the curvature of the wearer's mouth and gaps between the adjacent teeth. If the deformable strip of material includes the multi-phase oral care composition coated thereon, the multi-phase oral care composition may have an overall thickness less than about 1.5 mm. Deformable strips as disclosed herein may also be used as the material for the strip of material 12 shown in Figs. 1 to 4. Thus, general features of a strip of material as described above for example with respect to Figs. 1 to 4 may also apply to the deformable strip of material. In addition, a release liner and/or shallow pockets may also be combined with a deformable strip of material.

The present compositions may be used in combination with a delivery carrier including a dental tray and/or foam material. Suitable dental trays include rigid appliances, oversized rigid custom dental appliances rigid bilaminated custom dental appliances, disposable soft foam trays.

A rigid appliance which is fitted precisely to the patient's dental arches. For example, an alginate impression which registers all teeth surfaces plus gingival margin is made and a cast is promptly made of the impression. An "oversized" rigid custom dental appliance. The fabrication of rigid, custom dental appliances entails fabricating cast models of the patient's dental arch impressions, and heating and vacuum-forming a thermoplastic sheet to correspond to the cast models of a patient's dental arches. A third type of rigid custom dental appliance, used with less frequency, is a rigid bilaminated custom dental appliance fabricated from laminations of materials, ranging from soft porous foams to rigid, non-porous films. The non-porous, rigid thermoplastic shells of these bilaminated dental appliances encase and support an internal layer of soft porous foam.

Disposable U-shaped soft foam trays can be be individually packaged, and which may be saturated with a pre-measured quantity of the composition of the present invention. The soft foam material is generally an open celled plastic material. Such a device is commercially available from Cadco Dental Products in Oxnard, Calif. under the tradename VitalWhite^{™}.

In certain embodiments, the tray may have pockets built into the surface covering or contacting one or more teeth. Such pockets may help hold the oral composition in contact with the teeth. In certain embodiments, the pockets may be from about 0.05 to about 5 mm deep, preferably from about 0.1 to about 3mm deep, more preferably from about 0.3 to about 3 mm deep, or most preferably from about 0.5 to about 1.5 mm deep. Examples of such trays include those specified in the Clinical Protocol section.

In certain embodiments, the fit of the tray to the oral cavity may have a tolerance or gap built into it on the oral cavity. Such as tolerance or gap may help hold the oral composition in contact with the oral cavity. In certain embodiments, the tolerance or gap may be from about 0.01 mm to about 2 mm, preferably from about 0.05 mm to about 1 mm, more preferably from about 0.1 mm to about 1 mm, or most preferably from about 0.1 mm to about 0.5 mm.

### Product Forms

As described herein, the oral care composition can be a leave-on gel composition comprising mucoadhesive polymer to increase contact time between the gum health compound and/or amino acid with the gingiva. However, other products are also suitable either with or without a delivery carrier.

Suitable compositions for the delivery of the gum health compound and/or amino acid include emulsion compositions, such as the emulsions compositions of U.S. Patent Application Publication No. 2018/0133121, unit-dose compositions, such as the unit-dose compositions of U.S. Patent Application Publication No. 2019/0343732, jammed emulsions, dentifrice compositions, mouth rinse compositions, mouthwash compositions, tooth gel, subgingival gel, mouth rinse, mousse, foam, mouth spray, lozenge, chewable tablet, chewing gum, tooth whitening strips, floss and floss coatings, breath freshening dissolvable strips, denture care products, denture adhesive products, or combinations thereof.

### EXAMPLES

The invention is further illustrated by the following examples, which are not to be construed in any way as imposing limitations to the scope of this invention. Various other aspects, modifications, thereof which, after reading the description herein, may suggest themselves to one of ordinary skill in the art without departing from the scope of the appended claims.

### Skin Aging Study

150 Caucasian (Fitzpatrick I/II) women of different age groups were enrolled in the aging of skin study. The age groups included were 18-22 years old), 30s (30-34), 40s (40-44), 50s (50-54), 60s (60-64), 70s (70-74) & 80s (80-84) year olds.

A 4-mm skin punch biopsy was obtained from the buttocks by a single operator; snap-frozen in liquid nitrogen and stored at -80°C until assayed. The dermis was separated from the remaining portions of the biopsy by laser capture microdissection. To determine gene expression levels, RNA was extracted, labeled with biotin, hybridized, and analyzed using Affymetrix GeneTitan^{®} U219 microarray plates according to the manufacturer's instructions.

For genomics analysis, microarray sample quality was assessed using principal components analysis and MA-plots. Age group comparisons of 60 year old subjects vs 20 year old subjects were assessed using analysis of variance (ANOVA) models fitted separately for each probeset. Calculations were done in the limma R package. A p-value cutoff of 0.05 or less was consider statistically significant.

### Gingival Aging Study

Caucasian adults within the age ranges of 20 to 24 (20s), 30 to 34 (30s), 40 to 44 (40s), 50 to 54 (50s), 60 to 64 (60s), and 70 to 74 (70s), in good general health and oral health were enrolled. Eligible participants had at least 16 natural teeth and were free of erythematous or ulcerative conditions and periodontal disease with no pocket depth of >4 mm or generalized gingival inflammation.

A 2-mm gingival punch biopsy was obtained from the attached gingiva around the premolar teeth by a single operator; snap-frozen in liquid nitrogen and stored at -80°C until assayed. The frozen gingival tissue was then processed for gene expression analysis. To determine gene expression levels, RNA was extracted, labeled with biotin, hybridized and analyzed using Affymetrix GeneTitan^{®} U219 microarray plates according to the manufacturer's instructions.

For genomics analysis, microarray sample quality was assessed using principal components analysis and MA-plots. Age group comparisons of 60-year-old subjects vs 20-year-old subjects were assessed using analysis of variance (ANOVA) models fitted separately for each probeset. Calculations were done in the limma R package. A p-value cutoff of 0.05 or less was consider statistically significant.

### Compound Screening on Skin Dermis

To screen compounds, 30 to 40 female subjects with Fitzpatrick skin types I-III, aged 60-70. At Baseline, each subject was assigned to test each treatment. The products were applied to the dorsal forearm twice daily. Topical treatment with a minimally efficacious moisturizer was used as the vehicle for each test compound and as the negative control.

After four weeks of treatment, one 4-mm punch biopsy was taken from each treated site. The dermis was separated and snap-frozen in liquid nitrogen and stored at -80°C until assayed. The frozen tissue was then processed for gene expression analysis. To determine gene expression levels, RNA was extracted, labeled with biotin, hybridized and analyzed using Affymetrix GeneTitan^{®} U219 microarray plates according to the manufacturer's instructions.

For genomics analysis, microarray sample quality was assessed using principal components analysis and MA-plots. Treatment to negative control comparisons for the microarray data were assessed using ANOVA models fitted separately for each probe set. ANOVA model hypothesis tests were evaluated using the Empirical Bayes method available in the limma R package. A p-value cutoff of 0.05 or less was consider statistically significant.

### Results

A human clinical protocol for compound screening that tested for the expression of collagen genes after the application of materials to the skin. A retinoid (retinol at 0.2%), a palmitoyl pentapeptide (Pal-KTTKS at 100ppm), and the combination of the two were tested and compared to a vehicle control. A number of novel molecular similarities were observed in skin and gingival tissue aging as determined by Skin and Gingival Aging Studies. An analysis of gene expression and a comparison across the aging studies identified similar, statistically significant, decreasing patterns in important collagen genes as a function of age in both skin and gingival tissues. Specifically, an age-related decrease in both the major collagen genes, COL1A1 and COL1A2, as shown in TABLE 1.

A human clinical protocol for Compound Screening on Skin that tested for the expression of collagen genes after the application of materials to the skin. A retinoid (retinol at 0.2%), a palmitoyl pentapeptide (Pal-KTTKS at 100ppm), and the combination of the two were tested and compared to a vehicle control. Unexpectedly, COL1A1 and COL1A2 demonstrated statistically significantly increased gene expression with the combination of retinol and Pal-KTTKS but not with either retinol or Pal-KTTKS alone TABLE 1.

**TABLE 1. Regulation of collagen genes**

| Gene | Skin Age LFC (p-value) | Gingiva Age LFC (p-value) | Retinol LFC (p-value) | Pal-KTTKS LFC (p-value) | Retinol & Pal-KTTKS LFC (p-value) |
|---|---|---|---|---|---|
| COL1A1 | -0.99 (0.0012) | -0.66 (0.027) | -0.10 (0.93) | -0.15 (0.44) | 0.17 (0.037) |
| COL1A2 | -0.33 (0.0013) | -0.64 (0.0091) | -0.022 (0.88) | -0.048 (0.91) | 0.13 (0.043) |

TABLE 1 shows that the two primary collagen genes tested demonstrated significant (p-value <0.05) down regulation as a function of age in both skin and gingiva tissue. These collagen genes also demonstrated up-regulation upon treatment with retinol and Pal-KTTKS together (compared to vehicle treatment on the same subject). However, the collagen genes shown in TABLE 1 did not demonstrate significant regulation upon treatment with retinol or Pal-KTTKS separately, indicating a synergistic relationship amongst the retinoid and peptide. Regulation shown as Log base 2 of the average Fold Change (LFC) and p-values determined by ANOVA testing.

Based on the observations from the comparative skin and gingival aging gene expression studies in TABLE 1, and the hypothesis that collagen loss contributes to both skin and gingival aging, we conducted a human clinical study evaluating the effect of the combination of retinol and Pal-KTTKS when applied topically in a mucoadhesive gel. The cohort of 24 individuals from 34 to 65 showed varying recession of gingival margin from -0.51 to 0.76.

### Compound Assessment for Gingiva

Twenty-four subjects with two age related recessions were enrolled into a split mouth design, clinical study with 9 study visits. At Baseline Day 1 visit, subjects received oral soft tissue (OST) exam, digital gingival image (DGIA) and full periodontal exam (probing pocket depth (PPD), clinical attachment level (CAL) and recession (CEJ)). At Baseline Day 2 digital and traditional impressions of subjects' teeth were taken followed by Danser Exam (GA) of gum abrasion. Subjects' test teeth with recessions were randomized to treatment or placebo and subjects received products and were instructed to used it once a day for next six months. Subjects returned to the site after 1 month 2 months, 3 months, 4 months, 5 months and 6 months (Day 1 and Day 2 - final visits).

**TABLE 2. Repair of Gingival Recession in RCT (regeneration of gingival margin in mm)**

| **Analysis of Covariance for Selected Teeth - Buccal Sites** | | | | | | |
|---|---|---|---|---|---|---|
| | | **Month 6** | **Change from Baseline** | | **Treatment Difference** | |
| **Parameter** | **Treatment** | **Mean (SE)** | **Mean (SE)** | **P-value** | **Mean (SE)** | **P-value** |
| Gingival Recession | Ex. 12 | 0.278 (0.116) | 0.751 (0.116) | <0.0001 | | |
| | Ex. 11 Comparative | -0.070 (0.116) | 0.402 (0.116) | 0.0023 | -0.3487 (0.137) | 0.0193 |

The results show that after six months of treatment, the combination of retinol and Pal-KTTKS demonstrated statistically significant improvement in gingival recession by multiple measures when compared to the mucoadhesive vehicle alone (recession p = 0.019), improvements that were visibly observable, as shown in FIG. 4. Increased gingival barrier protection was also observed, as determined by reduced abrasion post brushing.

The improvement of gingival recession resulting from a topical treatment combining a retinoid and a peptide has not been previously demonstrated in a human clinical setting. Surprisingly, increased gingival resilience to damage was also observed, as determined by a reduction in post-toothbrushing abrasion.

### Test Methods

### Test 1: In-vitro human gingival fibroblasts soluble collagen assay

Stage 1: Culturing Primary Human Gingival Fibroblasts ("HGF"): Primary Human Gingival Fibroblasts ("HGF") were collected from tooth extraction patients and washed with 5 mL of PBS (phosphate buffered saline). The tissues were chopped into small pieces and placed into 15 mL centrifuge tube. The samples were digested with equal amounts of 1 mL 8% dispase and 1 mL 6% collagenase for 1 hr at 37 °C, during which time the samples need to be shaken every 15 minutes. Once the digestion process was complete, the tube was centrifuged at 1100 r.p.m. for 6 minutes at room temperature. After centrifugation, a pellet of cells was formed in the bottom of the tube separating them from the supernatant solution. Then the supernatant was discarded, and the cell pellet was suspended in 3 mL of fresh Minimum Essential Medium ("MEM", available from Thermo Fisher) culture medium then transferred to a petri dish. The petri dish with cells were placed in the incubator at 37 °C with 5% CO2 for about 10 days. The petri dish was checked for changes in medium color every two days. Fresh culture medium was replaced if changes in medium color occurred.

When there was 80-90% cell monolayer coverage of the petri dish, the present culture medium was removed and washed with 5 mL of PBS. 1 mL 0.25% trypsin-EDTA solution was added and the cells sat for about 1-2 minutes at 37 °C until the cells were visibly round-shaped. It was occasionally necessary to tap the petri dish to remove any sticky cells from the petri dish surface. At least 1 mL of fresh MEM culture medium was added to inactivate the trypsin and the cells are collected into a 15 mL centrifuge tube. The tube was then centrifuged at 1100 r.p.m. for 6 minutes at room temperature. The supernatant was discarded, and cell pellet is re-suspended in 4 mL of fresh MEM culture medium in the same centrifuge tube. Petri dishes were each placed with 1 mL cell suspension and 9 mL fresh MEM culture medium in the incubator at 37 °C with 5% CO2 for about 3-5 days until 80-90% cell monolayer coverage on the petri dishes was observed. Stage 2: Treatment with Compositions: When there was 80-90% cell monolayer coverage on the petri dishes, cells were re-suspended in 6 mL of fresh MEM culture medium. 1 mL cell suspension and 1 mL fresh MEM culture medium were respectively added into each well of a 6-well plate. The cells were further washed with 2 mL PBS to remove any suspended cells until no suspended cells are visible. 2 mL culture medium, or 2 mL culture medium containing 1% Comparative Compositions or 2 mL culture medium containing 1% Inventive Compositions were added to the wells. Cells were maintained in the incubator at 37 °C with 5% CO2 for 72 hours.

Stage 3: Soluble Collage Assay: Soluble collagen (types I to V) levels were measured by Sircol collagen assay kit (available from Biocolor, UK). After treatment, cells were washed two times with chilled PBS and homogenized with 6M urea. The cell homogenates were centrifuged at 15,000 r.p.m. for 15 min. 100 µL of the supernatant was mixed with 1 mL of Sircol Dye Reagent for 30 minutes. During this time period, a collagen-dye complex formed and precipitated out from the soluble unbound dye. The tubes were transferred to a microcentrifuge and spun at 12,000 r.p.m. for 10 minutes. The tubes were carefully inverted and drained. 750 µL ice-cold Acid-Salt Wash Reagent was gently added to the collagen-dye pellet to remove unbound dye from the surface of the pellet and the inside surface of the microcentrifuge tube. The tube was then centrifuged at 12,000 r.p.m. for 10 minutes. The supernatant was drained into a waste container and any fluid from the lip of the tubes was removed using cotton wool buds. 250 µL of Alkali Reagent was added to each tube to fully release the collagen bound dye. 200 µL of each sample was transferred to individual wells of a 96 micro well plate, keeping a record map of the contents of each well from A1 to H12. The microplate reader was set to 555 nm and absorbance against water was measured for the reagent Blanks, Standards and Test Samples. Collagen concentrations were obtained from the Standard Curve, which contained 5, 10, 15 and 20 µg of the Collagen Reference Standard in 100 µL fresh MEM culture medium. A p-value cutoff of 0.05 or less was consider statistically significant.

### Test 2: In-vitro 3D full-thickness gingival tissue model assay

*In-vitro* ginigival 3D tissue were used to assess the effects of treatment with Inventive Compositions and Control Compositions on histology and immune chemistry.

The initial preparation of the full-thickness gingival tissue model (GIN-300-FT, available from MatTek, US) was similar with methods previously described to produce other full thickness tissue models (Delvenne et al., 2001; Odioso et al., 1995). Briefly, 1 mL of serum containing 1 * 10⁶ gingival fibroblasts was mixed with a collagen solution to form a fibroblast-collagen gel matrix. The mixture was allowed to gelatinize by incubating the inserts at 37 °C for 1h. The gel was then equilibrated with 2 mL of growth medium and cultured for 48 h. Thereafter, autologous gingival epithelial cells were seeded atop the matrix and the tissue was cultured in incubator at 37 °C with 5% CO₂ for 2-3 weeks until stratum corneum at apical layer is observed, resulting in a multilayered *in vitro* human gingival culture derived from primary human gingival epithelial cells and fibroblasts.

Inventive Compositions and Comparative Compositions were diluted in sterile water to a 10% concentration, then the 10% slurries were sterile filtered by passing through filters with a pore size of 0.22 µL. 100 µL of each filtered 10% slurry was added to the apical side of the tissue. Tissues were cultured for 48 hours in incubator at 37 °C with 5% CO₂.

### Histology and Immunohistochemical analysis

To examine the morphology of the in vitro reconstructed tissues, inserts containing tissues were fixed with 10% formalin, embedded in paraffin, and 5-7 µm thick cross-sections were cut. The sections were mounted on microscopic slides, stained with hematoxylin and eosin (H&E, available from Sigma, US), and observed and photographed using a Nikon Diaphot microscope outfitted with a CoolPix 990 digital camera.

Tissues were fixed in Carnoy's fixative solution (acetic acid: ethanol: chloroform, 10:60:30) overnight at 4 °C, embedded in paraffin, and sectioned for routine histology. Sections were deparaffinized and rehydrated. Endogenous peroxide was blocked using 1% H₂O₂/Tris buffered saline (TBS). After blocking with 3% normal serum of the source of secondary biotinylated antibody, the sections were incubated with primary antibodies. Detection was via the avidinbiotin-peroxidase complex method using diaminobenzidene as substrate. A p-value cutoff of 0.05 or less was considered statistically significant. The antibodies used are Collagen-1, Elastin and Fibrillin-1, as shown in TABLE 3, below.

**TABLE 3. Antibodies**

| **Target** | **1° antibody** | **Cat#** | **Company** |
|---|---|---|---|
| Collagen-1 | Collagen I alpha 1 antibody | NBP1-82488 | Novus Biologicals |
| Elastin | Anti-Elastin Antibody | AB2043 | Millipore |
| Fibrillin-1 | Anti-FBN1 | AMAb90585 | Atlas antibodies |
| Actin | Anti-actin antibody | AB179467 | AbCam |
| E-Cadherin | mAb to E-cadherin [HECD-1] | Ab1416 | AbCam |
| Claudin | Anti-Claudin 1 antibody | Ab15098 | AbCam |

### Test 3: Muco-adhesion Test Method

Test samples were labeled with Fluorescein isothiocyanate (FITC) at room temperature for 1 hour. The resulting solutions were dialyzed in phosphate buffered saline (PBS) for 48 hr in order to remove the free FITC molecules. The silica wafers were treated with a mixture of ethanol/(3-Aminopropyl) triethoxysilane (APTES)/ammonia solution (20:4:1, v/v/v) for 8 hours and were then rinsed with ethanol and water, and dried to obtain the amine-functionalized silica wafers (NH₂-SW). NH₂-SW were incubated in mucin solution for 8 hours at room temperature. The mucin modified silica wafers were incubated with test sample in a PBS buffer in a shaker at 37°C for 12 hours followed by rinsing with deionized water. The fluorescence images of the mucin modified silica wafers were taken by a fluorescence microscopy and used for measurement of Fluorescent Intensity (FI) in ImageJ software (National Institutes of Health, USA, (https://imagej.nih.gov/ij/)). The Fluorescent Intensity Percentage ("FI%"), which was used to describe the "Mucoadhesion Index", was the fluorescent pixels relative to total pixels in a normalized image field. Therefore, a first test sample having a higher FI% had stronger mucoadhesion compared to a second test sample having a lower FI%.

### Test 4: Rheological Test Method

A Rheological Test Method is described for assessing viscosity profile (and ran according to manufacturing instructions). The viscosity profiles were tested on a TA AR2000 rheometer (available from TA Instruments, New Castle, United States) by using Dentifrice Macro Rheology Test procedure. The geometry used was 40 mm steel parallel plate with solvent trap. Dentifrice was placed on the Peltier Plate of AR2000 rheometer and the Gap setting was 1000 micron. Dentifrice Macro Rheology Test included stress sweep oscillation, frequency sweep oscillation and steady state flow tests. The key parameter settings included: (a) Stress sweep step: Oscillation Stress (Pa): 0.01 - 500; (b) Frequency (Hz): 1.0; (c) Frequency sweep step: Frequency (Hz): 0.1 - 10, and Controlled Oscillation Stress (Pa): 1.5; (d) Steady state flow step 1: Shear rate (s⁻¹): 0.01 - 100; and (e) Steady state flow step 2: Shear rate (s⁻¹): 100 - 0.01.

Shear flow test is a viscosity testing mode to measure the viscosity at different shear rates. Steady state flow test is a flow in which the velocity at every point does not vary with time. The three main parameters in this test were viscosity, shear rate and shear stress. Power Law Model is a well-known model used to characterize the relationship between viscosity or shear stress and shear rate over the range of shear rates where shear thinning occurs in a Non-Newtonian fluid. It quantified overall viscosity range and degree of deviation from Newtonian behavior. The Power Law Model is described as ***η*= *Kγ*^{*n*-1},** wherein ***η*** = viscosity and ***γ*** = shear rate. ***K*** is known as the Viscosity Consistency Coefficient, describing the overall range of viscosities across the part of the flow curve that is being modelled. The exponent *"n"* is known as the rate index. *K* and ***η*** can be determined by Power Law Model fitting. Based upon rate index n, the Power Law Model describes three basic types of flow:
- *n*=1: Newtonian behavior
- *n<*1: Shear thinning (or Pseudoplastic)
- *n>*1: Shear thickening

The viscosity profiles of the composition herein are represented by the Viscosity Consistency Coefficient *K* (Pa·s) determined by Power Law Model fitting with the shear rate range of 0.1-10 s⁻¹.

### Test 5: Assay for Measuring Improved Wound Healing of Human Gingival Fibroblasts

*In-vitro* human gingival fibroblasts were used to assess the effects of wound healing migration as a result of treatment with Inventive Compositions and Control Compositions. The method involves three stages:
Stage 1: Culturing Primary Human Gingival Fibroblasts ("HGF"): Human gingival fibroblasts were collected from tooth extraction patients and washed with 5 mL of PBS (phosphate buffered saline). The tissues were chopped into small pieces and placed into 15 mL centrifuge tube. The samples were digested with equal amounts of 1 mL 8% dispase and 1 mL 6% collagenase for 1 hr at 37 °C, during which time the samples needed to be shaken every 15 minutes. Once the digestion process was complete, the tube was centrifuged at 1100 RPM for 6 minutes at room temperature. After centrifugation, a pellet of cells was formed in the bottom of the tube separating them from the supernatant solution. Then the supernatant was discarded and the cell pellet was suspended in 3 mL of fresh Minimum Essential Medium ("MEM", available from Thermo Fisher) culture media then transferred to a petri dish. The petri dish with cells were placed in the incubator at 37 °C with 5% CO₂ for about 10 days. The petri dish was checked for changes in media color every two days. Fresh culture media was replaced if changes in media color occurred.
Stage 2: Sub-Culturing Human Gingival Fibroblast: When there was 80-90% cell monolayer coverage of the petri dish, then the present culture media was removed and washed with 5 mL of PBS. 1 mL 0.25% trypsin-EDTA solution was added and the cells sit for about 1-2 minutes at 37 °C until the cells were visibly round-shaped. It was occasionally necessary to tap the petri dish to remove any sticky cells from the petri dish surface. At least 1 mL of fresh MEM culture media was added to inactivate the trypsin and the cells were collected into a 15 mL centrifuge tube. The tube was then centrifuged at 1100 RPM for 6 minutes at room temperature. The supernatant was discarded and cell pellet was re-suspended in 4 mL of fresh MEM culture media in the same centrifuge tube. 4 petri dishes were each placed with 1 mL cell suspension and 9 mL fresh MEM culture media in the incubator at 37 °C with 5% CO₂ for about 3-5 days until 80-90% cell monolayer coverage on the petri dishes were observed. This stage was repeated 2-4 times before the wound healing assay to achieve the highest cell viability.
Stage 3: Wound Healing Assay: When there was 80-90% cell monolayer coverage on the petri dishes, the present culture media was removed and washed with 5 mL of PBS. 1 mL 0.25% trypsin-EDTA solution was added and the cells sat for about 1-2 minute at 37 °C until the cells were visibly round-shaped. It was occasionally necessary to tap the culture petri dish to remove any sticky cells from the petri dish surface. At least 1 mL of fresh MEM culture media was added to inactivate the trypsin and the cells were collected into a 15 mL centrifuge tube. The tube was then centrifuged at 1100 RPM for 6 minutes at room temperature. The supernatant was discarded and cell pellet is re-suspended in 6 mL of fresh MEM culture media. 1 mL cell suspension and 1 mL fresh MEM culture media were respectively added into each well of a 6-well plate. The plates were incubated at 37 °C with 5% CO₂ until 50-70% cell monolayer coverage is formed. The outer bottoms of wells were then marked with a line in middle as the reference line during image acquisition. A wound was created manually by scraping the right half of cell monolayer with a sterilized 1 mL pipette tip. The cells were washed with 2 mL PBS to remove any suspended cells until no suspended cells were visible. 2 mL culture media, and 2 ml culture media containing 1% Control Compositions or 2 mL culture media containing 1% Inventive Compositions were added to the wells.

High density digital images of the HGF were captured with an Olympus^{®} IX71 digital SLR camera with an Olympus^{®} UIS2 WHN10X objective lens. The first images were acquired at time 0 hr (*i.e.,* Baseline) by using the middle line markings on the plates as a reference line. The plates were then incubated at 37 °C with 5% CO₂ for varying time intervals as described below. The matched photographed region was acquired as previously, and images were acquired at later time intervals (*e.g*., 16 hr, 24 hr, 48 hr, 65 hr, 72 hr, etc.) after baseline to assess the cell coverage (%) as an indication of the wound healing performance under the different treatment legs. Images were evaluated by Wimasis^{®} WimScratch software (available from Wimasis GmbH, Germany) to determine the degree (*i.e.,* percentage) of HGF cell coverage (*i.e.,* wound healing) pass the marked wound boundary, as indicated by the dotted line, as compared to the matching baseline image for each sample. WimScratch software utilized advanced edge detection and overlay techniques to recognize cells and blank area, *i.e.* the green overlay in the image represents the cell-covered area of the particular image and the grey area represents the wound area. The readout is presented for both area and is normalized as percent of total area.

### Test 6: Assay for Measuring Barrier Permeability of Human Gingival Epithelia

*In-vitro* human gingival epithelia were used to assess effects of barrier permeability as a result of treatment with Inventive Compositions and Control Compositions. The method involves three stages:
Stage 1: Culturing Primary Human Gingival Epithelia ("HGE"): Human gingival epithelia were collected from tooth extraction patients and washed with 5 mL of PBS (phosphate buffered saline, available from Sigma). The tissues were chopped into small pieces and placed into 15 mL centrifuge tube. The samples were digested with equal amounts of 1 mL 8% dispase and 1 mL 6% collagenase for 1 hour at 37 °C, during which time the samples need to be shaken every 15 minutes. Once the digestion process is complete, the tube was centrifuged at 1100 RPM for 6 minutes at room temperature. After centrifugation, a pellet of cells was formed in the bottom of the tube separating them from the supernatant solution. Then the supernatant was discarded and the cell pellet is suspended in 3 mL of fresh Dulbecco's Minimum Essential Medium ("DMEM", available from Thermo Fisher^{®}) culture media then transferred to a petri dish. The petri dish with cells were placed in the incubator at 37 °C with 5% CO₂ for about 10 days until 80-90% cell monolayer coverage on the petri dishes was observed. Before the assay started, HGE cells were seeded (1.2*10⁴ cells/well) on hanging cell culture inserts (Merck Millipore, Billerica, MA, USA; 5 um pore size) placed in 24-well plastic tissue-culture plates (Merck Millipore) and grown in fresh DMEM culture media at 37 °C with 5% CO₂ for 72 hours until they reached confluence. The volumes of the medium in the upper compartment and lower compartment were 0.2 mL and 0.8 mL, respectively.
Stage 2: Treatment by Compositions: The HGE cell monolayer were incubated for another 72 h in fresh DMEM culture media in the presence or absence of 10 ug/mL P. gingivalis-LPS (available from Invivogen, CA, US), Inventive Compositions (1% gel in DMEM culture media, w/w) and Comparative Compositions (1% gel in DMEM culture media, w/w). Then the medium was removed from both compartments, and the upper compartments were washed with 0.2 mL PBS for twice and placed in fresh new lower compartments.
Stage 3: Measurement of Epithelial Barrier Permeability: To examine cell permeability of a common pathogen in dentistry, P. gingivalis- LPS was used as a transport substance. 0.2 mL fresh DMEM culture media containing 10 ug/mL P. gingivalis-LPS was added to the upper compartments, and 0.8 mL fresh DMEM culture media without P. gingivalis-LPS was added to the lower compartments. The hanging cell culture inserts with cells are placed in the incubator at 37 °C with 5% CO₂ for 4 hours after the addition of P. gingivalis-LPS. The medium was collected from the lower compartment at 4 hour time point. Endotoxin units in the collected medium were measured by the Limulus Amebocyte Lysate (LAL) test (Associates of Cape Cod, Inc., Falmouth, MA, USA) according to manufacturer's instructions.

**TABLE 4: Oral Composition Formulations**

| Ingredients | Ex.1 | Ex.2 | Ex. 3 | Ex. 4 | Ex. 5 | Ex. 6 | Ex. 7 | Ex. 8 | Ex. 9 | Ex.10 |
|---|---|---|---|---|---|---|---|---|---|---|
| Glycerin | 20.00 | 20.000 | 20.000 | 20.000 | 20.000 | 20.000 | 20.000 | 20.000 | 20.000 | 20.000 |
| Xylitol | 2.000 | 2.000 | 2.000 | 2.000 | 2.000 | 2.000 | 2.000 | 2.000 | 2.000 | 2.000 |
| PEG 300 | 0.500 | 0.500 | 0.500 | 0.500 | 0.500 | 0.500 | 0.500 | 0.500 | 0.500 | 0.500 |
| Mucoadhesive Polymers (Carbomer, PVPK90, Hyaluronate) | 3.526 | 3.526 | 3.526 | 3.526 | 3.526 | 3.526 | 3.526 | 3.526 | 3.526 | 3.526 |
| Retinoic Acid | - | - | - | 0.002 | - | - | - | - | - | - |
| Retinol | - | 0.002 | - | - | - | - | - | 0.15 | - | 0.15 |
| Retinyl Palmitate | - | - | 0.002 | - | - | - | - | - | - | |
| pal-KTTKS peptide | - | - | - | - | 0.001 | - | - | | 0.003 | 0.0036 |
| GHK peptide | - | - | - | - | - | 0.001 | - | | - | - |
| GEKG peptide | - | - | - | - | - | - | 0.001 | - | - | - |
| Sodium Pyrophosphate | 0.500 | 0.500 | 0.500 | 0.500 | 0.500 | 0.500 | 0.500 | 0.500 | 0.500 | 0.500 |
| Sodium Hydroxide (50%) | 0.900 | 0.900 | 0.900 | 0.900 | 0.900 | 0.900 | 0.900 | 0.900 | 0.900 | 0.900 |
| Preservatives | 0.150 | 0.150 | 0.150 | 0.150 | 0.150 | 0.150 | 0.150 | 0.150 | 0.150 | 0.150 |
| Flavorant | 1.030 | 1.030 | 1.030 | 1.030 | 1.030 | 1.030 | 1.030 | 1.030 | 1.030 | 1.030 |
| Sodium Saccharin | 0.100 | 0.100 | 0.100 | 0.100 | 0.100 | 0.100 | 0.100 | 0.100 | 0.100 | 0.100 |
| Water and minors (e.g. coloring agent) | Q.S. | Q.S. | Q.S. | Q.S. | Q.S. | Q.S. | Q.S. | Q.S. | Q.S. | Q.S. |

**TABLE 5: Effect of Retinoid to upregulate in vitro collagen synthesis**

| | Total Collagen (ug/100ul) | Total Cell Count Cell (×10⁵) | Collagen Productivity (Collagen/Cell) | Upregulation of Collagen Synthesis vs. Baseline |
|---|---|---|---|---|
| Baseline Control | 10.00 | 2.65 | 3.77 | - |
| Positive Control (TGF-B, 5 ppm) | 13.44 | 2.60 | 5.17 | - |
| Ex. 2 | 19.09 | 2.67 | 7.07 | 87.5% |
| Ex. 3 | 16.25 | 2.73 | 5.94 | 57.6% |

The results in TABLE 4 and 5 show that the Inventive Composition Ex. 2 & Ex 3. comprising the Gum Health compound (Retinoids at 20ppm), i.e. retinol and retinyl palmitate respectively, effectively upregulates the collagen synthesis at 87% and 57% collagen productivity vs. base control. Ex. 2 and 3 even outperformed the positive control TGF-B.

**TABLE 6: Effect of peptide to upregulate in vitro collagen synthesis**

| (1:100 dilution) | Total Collagen (ug/100ul) | Total Cell Count Cell (×10⁵) | Collagen Productivity (Collagen/Cell) | Upregulation of Collagen Synthesis vs. Baseline |
|---|---|---|---|---|
| Baseline Control | 10.00 | 2.65 | 3.77 | - |
| Positive Control (TGF-B, 5 ppm) | 13.22 | 2.72 | 4.86 | - |
| Ex. 5 | 12.99 | 2.60 | 4.99 | 32.3% |
| Ex. 6 | 14.42 | 2.57 | 5.60 | 48.5% |
| Ex. 7 | 16.37 | 2.56 | 6.34 | 68.2% |

The results show that the Inventive Composition Ex. 5, Ex. 6, and Ex. 7 containing peptide at 10ppm, i.e. pal-KTTKS, GHK, GEKG respectively, effectively upregulates the collagen synthesis at 14%, 48%, 68% collagen productivity vs. vs. base control and positive control TGF-B.

**TABLE 7: In vitro upregulation of collagen synthesis**

| (1:100 dilution) | Total Collagen (ug/100ul) | Total Cell Count Cell (×10⁵) | Collagen Productivity (ug per 10⁵ cells / 100ul) | Upregulation of Collagen Synthesis vs. Baseline |
|---|---|---|---|---|
| Blank Control | 10.54 | 2.75 | 3.83 | - |
| Positive Control (TGF-B, 5 ppm) | 13.24 | 2.72 | 4.86 | - |
| Ex. 8 (retinol) | 12.99 | 3.19 | 4.07 | 6.3% |
| Ex. 9 (pal-KTTKS) | 12.76 | 3.09 | 4.13 | 7.8% |
| Ex.10 (retinol + pal-KTTKS) | 16.55 | 2.64 | 6.27 | 63.7% |

TABLE 7 shows the unexpected result that the combination of retinoid compound and peptide improves collagen productivity. Surprisingly, the increase in collagen productivity vs. base control was significantly higher in the Ex. 10 for the combination of retinol & pal-KTTKS (2.44) than the sum of the individuals (pal-KTTKS = (0.24) and Retinol (0.30) vs. the base control). This results in TABLE 7 indicated that the combination of gum health compound and amino acid, and/or more specifically retinoid compound and peptide provides an unexpectedly high improvement in collagen upregulation relative to the sole ingredients.

**TABLE 8: Oral Composition Formulations**

| Ingredients | Ex.11 Comparative | Ex. 12 | Ex. 13 | Ex. 14 | Ex. 15 | Ex. 16 | Ex. 17 |
|---|---|---|---|---|---|---|---|
| Glycerin | 20.000 | 20.000 | 20.000 | 20.000 | 20.000 | 20.000 | 20.000 |
| Xylitol | 2.000 | 2.000 | 2.000 | 2.000 | 2.000 | 2.000 | 2.000 |
| PEG 300 | 0.500 | 0.500 | 0.500 | 0.500 | 0.500 | 0.500 | 0.500 |
| Mucoadhesive Polymers (Carbomer, PVPK90, Hyaluronate) | 3.526 | 3.526 | 3.000 | 3.526 | 3.526 | 3.526 | 3.526 |
| Allantoin | 0.100 | - | - | - | 0.100 | 0.100 | - |
| Panthenol | 0.500 | - | - | - | 0.500 | 0.500 | - |
| Tocopherol Acetate | 0.500 | - | - | - | 0.500 | 0.500 | - |
| Sodium Ascorbyl Phosphate | 0.910 | - | - | - | 0.910 | 0.910 | - |
| Niacinamide | - | - | - | - | - | - | 0.500 |
| Retinol | - | 0.150 | 0.150 | 0.150 | 0.150 | 0.150 | 0.150 |
| pal-KTTKS peptide | - | 0.003 | 0.003 | | 0.003 | - | 0.003 |
| Glycine | - | - | - | 0.5 | - | - | - |
| Sodium Pyrophosphate | 0.500 | 0.500 | 0.500 | 0.500 | 0.500 | 0.500 | 0.500 |
| Sodium Hydroxide (50%) | 0.900 | 0.900 | 0.900 | 0.900 | 0.900 | 0.900 | 0.900 |
| Preservatives | 0.150 | 0.150 | 0.150 | 0.150 | 0.150 | 0.150 | 0.150 |
| Flavorant | 1.030 | 1.030 | 1.030 | 1.030 | 1.030 | 1.030 | 1.030 |
| Sodium Saccharin | 0.100 | 0.100 | 0.100 | 0.100 | 0.100 | 0.100 | 0.100 |
| Water and minors (e.g. coloring agent) | Q.S. | Q.S. | Q.S. | Q.S. | Q.S. | Q.S. | Q.S. |

**TABLE 9: In vitro upregulation of collagen synthesis**

| (1:50 dilution) | Total Collagen (ug/100ul) | Total Cell Count Cell (×10⁵) | Collagen Productivity (Collagen/Cell) | Upregulation of Collagen Synthesis vs. Baseline |
|---|---|---|---|---|
| Blank Control | 10.54 | 2.18 | 4.83 | - |
| Positive Control (TGF-b, 5ppm) | 13.44 | 2.60 | 5.17 | - |
| Ex. 12 | 20.92 | 3.34 | 6.26 | 29.6% |
| Ex. 13 | 22.71 | 3.86 | 5.88 | 21.7% |
| Ex. 14 | 17.11 | 3.26 | 5.25 | 8.7% |

TABLE 9 shows that the Inventive Composition Ex. 12, Ex. 13, and Ex. 14 containing gum health compounds all effectively upregulates the collagen synthesis at 30%, 22%, 9% collagen productivity vs. base control.

Ex. 12 only differs from Ex. 13 based on the addition of hyaluronic acid to Ex. 12 relative to Ex. 13. Surprisingly, the addition of hyaluronic acid in Ex. 12 compared to Ex 13, increases the collagen productivity. It is hypothesized, but not wishing to be bound to the theory, the addition of hyaluronic acid controls the diffusion rate of the Gum health compound, in this case retinol, while also providing the mucoadhesive properties of hyaluronic acid. The control of the high retinol concentrations can reduce the irritating and ultimately toxic effects of retinoids, that are shown to activate TRP receptors, associated with irritation and negative sensory.

It was further surprising that Ex. 14 that used the combination of retinol and amino acid, glycine, increased the collagen productivity, and could be seen as alternative to peptides, based on molecular size may have improved diffusion and cost advantages vs. peptides.

**TABLE 10: 3D Human gingival tissue Immunohistochemical Response**

| | Epidermis | | | | Epidermal/Dermal | |
|---|---|---|---|---|---|---|
| Blank Untreated | Thickness | Actin | Claudin | E-Cadherin | Fibrillin | Collagen-1 |
| | 165 | 54.6 | 5.3 | 16.5 | 30.2 | 35.9 |
| Ex. 11 Comparative | 184 | 103.9 | 7.2 | 29.4 | 32.1 | 117.9 |
| Ex. 12 | 198 | 59.0 | 5.4 | 19.4 | 60.6 | 143.5 |
| Ex. 15 | 215 | 105 | 6.3 | - | - | 150.1 |
| Ex. 16 | 207 | 65 | 6.2 | - | - | 130.2 |

TABLE 10 shows the Inventive composition Ex. 12 containing gum health compounds retinol & peptide effectively delivers gum health compounds to the epidermal/dermal regions to upregulate the collagen-I and fibrillin production by 22% and 89% vs. the comparative Ex. 11. However, the inventive composition Ex. 12, containing retinol and peptide did not improve the epidermal immunochemical proteins measured in actin, claudin or e-cadherin, indicating no improvement in barrier functions properties vs. the comparative Ex. 11. Surprisingly, the addition of other gum health compounds, inventive composition Ex. 15 (Vitamin C, Vitamin E and Vitamin B6) with retinol and peptide, not only improved the collagen production, but also upregulated the barrier function properties of the 3D tissue to provide a much stronger and resilient 3D structure.

**TABLE 11: in-vitro human gingival fibroblast wound healing recovery**

| Healing Recovery | Cell Wound Healing Rate (%) @ 48hrs |
|---|---|
| Blank Control (Natural Healing) | 8.93 |
| Ex. 11 Comparative | 12.58 |
| Ex. 12 | 7.20 |
| Ex. 16 | 26.60 |
| Ex. 17 | 26.20 |

**TABLE 12: in-vitro gum gingival epithelia pemeability and barrier resilience from LPS challenge**

| Barrier Function (Transwell LPS Permeability) | LPS Challenge | LPS Permeability (LPS EU/ul) |
|---|---|---|
| Blank LPS Unchallenged | - | 2.72 |
| Blank LPS Challenged | + | 8.99 |
| Ex. 11 Comparative | + | 5.42 |
| Ex. 12 | + | 4.28 |
| Ex. 16 | + | 2.37 |
| Ex. 17 | + | 2.84 |

TABLE 11 & 12 shows that the Inventive Composition Ex. 16 & Ex. 17 that contain a combination of gum compounds which upregulate both the epidermis and epidermal/dermis proteins can further enhance the wound healing recovery and barrier function resilience and protection. This is shown in inventive composition Ex 16 (retinol, peptide and Vitamin A, B5, E) and Ex. 17 (retinol and Vitamin B3) vs. comparative Ex. 11, whereby the wound healing recovery improved 111%, 108% respectively and 56% and 47% improvement for the resilience to LPS permeability. Whereas in Ex. 12 (retinol and peptide only) there was no improvement in wound healing recovery and 21% improvement in resilience to LPS permeation.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

## Claims

1. A leave-on oral care composition comprising:
(a) gum health compound, preferably wherein the gum health compound comprises metal ion source, vitamin, allantoin, gum strengthening compound, or combinations thereof; and
(b) amino acid, wherein the amino acid comprises one or more amino acids, peptide and/or polypeptide and
wherein the gum health compound comprises retinol and the amino acid comprises pal-KTTKS,
preferably wherein the oral care composition is free of abrasive, or more preferably wherein the gum health compound is free of jasmonic acid compound, gibberellic acid, zeatin compound, or combinations thereof.

2. The oral care composition of claim 1, wherein the metal ion source comprises tin, zinc, copper, or combinations thereof, preferably wherein the tin comprises stannous fluoride, stannous chloride, or combinations thereof.

3. The oral care composition of claim 1 or 2, wherein the vitamin comprises vitamin A, vitamin B, vitamin C, vitamin D, vitamin E, derivatives thereof, or combinations thereof, preferably wherein the vitamin comprises retinoid compound, more preferably wherein the retinoid compound comprises vitamin A, a vitamer of vitamin A, a derivative of vitamin A, or combinations thereof, or even more preferably wherein the retinoid compound comprises retinyl ester, retinal, retinoic acid, tocopheryl-retinoate, tocopherol ester of *cis-* or *trans*-retinoic acid, isotretinoin, alitretinoin, etretinate, acitretin, adapalene, bexarotene, tazarotene, or combinations thereof.

4. The oral care composition of claim 3, wherein the retinyl ester comprises retinyl palmitate, retinyl acetate, retinyl propionate, or combinations thereof.

5. The oral care composition of any one of claims 1 to 4, wherein the amino acid comprises basic amino acid, neutral amino acid, acidic amino acid, or combinations thereof, preferably wherein the amino acid comprises naturally occurring amino acid, synthetic amino acid, and combinations thereof, or more preferably wherein the amino acid comprises arginine, histidine, lysine, aspartic acid, glutamic acid, serine, threonine, asparagine, glutamine, cysteine, selenocysteine, glycine, proline, alanine, valine, isoleucine, leucine, methionine, phenylalanine, tyrosine, tryptophan, citrulline, ornithine, creatine, diaminobutonic acid, diaminoproprionic acid, salts thereof, or combinations thereof.

6. The oral care composition of any one of claims 1 to 5, wherein the amino acid comprises peptide, polypeptide, or combinations thereof, preferably wherein the peptide comprises from two amino acids to ten amino acids, or more preferably wherein the peptide comprises a dipeptide, a tripeptide, a tetrapeptide, a pentapeptide, or combinations thereof.

7. The oral care composition of claim 6, wherein the peptide comprises lysine, threonine, serine, glycine, glutamic acid, histidine, proline, or combinations thereof, preferably wherein the peptide comprises a peptide with a sequence of glycine-histidine-lysine, glycine-glutamic acid-lysine-glycine.

8. The oral care composition of any one of claims 1 to 7, wherein the composition has a Viscosity Consistency Coefficient K of from 20 Pa·s to 500 Pa·s as measured at 22°C at a shear rate range of 0.1-10s⁻¹, preferably wherein the oral care composition has a Mucoadhesion Index of no less than 0.3 Fluorescent Intensity Percentage ("FI%"), more preferably wherein the composition comprises mucoadhesive polymer, or even more preferably wherein the mucoadhesive polymer comprises polyacrylic acid, natural gum, linear sulfated polysaccharide, anionic cellulose, nonionic cellulose derivative, polyvinyl pyrrolidine, hyaluronic acid, or combinations thereof.

9. The oral care composition of any one of claims 1 to 8, wherein the composition comprises fluoride, preferably wherein the fluoride comprises stannous fluoride, sodium fluoride, sodium monofluorophosphate, amine fluoride, or combinations thereof.

10. The oral care composition of any one of claims 1 to 9, wherein the gum health compound comprises pharmaceutical grade retinol.

11. The oral care composition of any one of claims 1 to 10 for use in treatment of gingival or gum recession comprising preventing gingival recession, stopping gingival recession, reversing gingival recession, decreasing gum recession, promoting collagen synthesis, promoting extracellular matrix synthesis, increasing gum resilience, increasing epidermal thickness, promoting fibrillin synthesis, or combinations thereof in an oral cavity of a user or an animal comprising contacting the oral care composition of any of claims 1 to 10 with at least one surface of the oral cavity.

12. A kit comprising:
(a) the leave-on oral care composition of any one of claims 1 to 10; and
(b) a delivery carrier.

13. The kit of claim 12, wherein the delivery carrier comprises strip, film of material, dental tray, aligner, sponge material, applicator, or mixtures thereof.

## Patentansprüche

1. Zum Belassen in dem Mund bestimmte Mundpflegezusammensetzung, umfassend:
(a) eine Verbindung für eine Zahnfleischgesundheit, vorzugsweise wobei die Verbindung für die Zahnfleischgesundheit eine Metallionenquelle, ein Vitamin, Allantoin, eine Verbindung für eine Zahnfleischstärkung oder Kombinationen davon umfasst; und
(b) eine Aminosäure, wobei die Aminosäure eine oder mehrere Aminosäuren, Peptid und/oder Polypeptid umfasst und
wobei die Verbindung für die Zahnfleischgesundheit Retinol umfasst und die Aminosäure pal-KTTKS umfasst,
vorzugsweise wobei die Mundpflegezusammensetzung frei von Schleifmitteln ist, oder mehr bevorzugt wobei die Verbindung für die Zahnfleischgesundheit frei von Jasmonsäureverbindungen, Gibberellinsäure, Zeatinverbindung oder Kombinationen davon ist.

2. Mundpflegezusammensetzung nach Anspruch 1, wobei die Metallionenquelle Zinn, Zink, Kupfer oder Kombinationen davon umfasst, vorzugsweise wobei das Zinn Zinnfluorid, Zinn(II)-chlorid oder Kombinationen davon umfasst.

3. Mundpflegezusammensetzung nach Anspruch 1 oder 2, wobei das Vitamin Vitamin A, Vitamin B, Vitamin C, Vitamin D, Vitamin E, Derivative davon oder Kombinationen davon umfasst, vorzugsweise wobei das Vitamin eine Retinoidverbindung umfasst, mehr bevorzugt wobei die Retinoidverbindung Vitamin A, ein Vitamer von Vitamin A, ein Derivat von Vitamin A oder Kombinationen davon umfasst, oder noch mehr bevorzugt wobei die Retinoidverbindung Retinylester, Retinal, Retinoinsäure, Tocopheryl-Retinoat, Tocopherolester von cis- oder trans-Retinsäure, Isotretinoin, Alitretinoin, Etretinat, Acitretin, Adapalen, Bexaroten, Tazaroten oder Kombinationen davon umfasst.

4. Mundpflegezusammensetzung nach Anspruch 3, wobei der Retinylester Retinylpalmitat, Retinylacetat, Retinylpropionat oder Kombinationen davon umfasst.

5. Mundpflegezusammensetzung nach einem der Ansprüche 1 bis 4, wobei die Aminosäure eine basische Aminosäure, eine neutrale Aminosäure, eine saure Aminosäure oder Kombinationen davon umfasst, vorzugsweise wobei die Aminosäure eine natürlich vorkommende Aminosäure, eine synthetische Aminosäure und Kombinationen davon umfasst, oder mehr bevorzugt wobei die Aminosäure Arginin, Histidin, Lysin, Asparaginsäure, Glutaminsäure, Serin, Threonin, Asparagin, Glutamin, Cystein, Selenocystein, Glycin, Prolin, Alanin, Valin, Isoleucin, Leucin, Methionin, Phenylalanin, Tyrosin, Tryptophan, Citrullin, Ornithin, Kreatin, Diaminobutonsäure, Diaminopropionsäure, Salze davon oder Kombinationen davon umfasst.

6. Mundpflegezusammensetzung nach einem der Ansprüche 1 bis 5, wobei die Aminosäure ein Peptid, ein Polypeptid oder Kombinationen davon umfasst, vorzugsweise wobei das Peptid von zwei Aminosäuren bis zehn Aminosäuren umfasst oder mehr bevorzugt wobei das Peptid ein Dipeptid, ein Tripeptid, ein Tetrapeptid, ein Pentapeptid oder Kombinationen davon umfasst.

7. Mundpflegezusammensetzung nach Anspruch 6, wobei das Peptid Lysin, Threonin, Serin, Glycin, Glutaminsäure, Histidin, Prolin oder Kombinationen davon umfasst, vorzugsweise wobei das Peptid ein Peptid mit einer Sequenz von Glycin-Histidin-Lysin, Glycin-Glutaminsäure-Lysin-Glycin umfasst.

8. Mundpflegezusammensetzung nach einem der Ansprüche 1 bis 7, wobei die Zusammensetzung einen Viskositätskonsistenzkoeffizienten K von 20 Pa·s bis 500 Pa·s aufweist, gemessen bei 22 °C bei einem Scherratenbereich von 0,1-10s⁻¹, vorzugsweise wobei die Mundpflegezusammensetzung einen Mucoadhäsionsindex von nicht weniger als 0,3 Fluoreszenzintensitätsprozentsatz ("FI%") aufweist, mehr bevorzugt wobei die Zusammensetzung ein mucoadhäsives Polymer umfasst, oder noch mehr bevorzugt wobei das mucoadhäsive Polymer Polyacrylsäure, natürlichen Gummi, lineares sulfatiertes Polysaccharid, anionische Cellulose, nichtionisches Cellulosederivat, Polyvinylpyrrolidin, Hyaluronsäure oder Kombinationen davon umfasst.

9. Mundpflegezusammensetzung nach einem der Ansprüche 1 bis 8, wobei die Zusammensetzung Fluorid umfasst, vorzugsweise wobei das Fluorid Zinnfluorid, Natriumfluorid, Natriummonofluorphosphat, Aminfluorid oder Kombinationen davon umfasst.

10. Mundpflegezusammensetzung nach einem der Ansprüche 1 bis 9, wobei die Verbindung für die Zahnfleischgesundheit Retinol in Arzneimittelqualität umfasst.

11. Mundpflegezusammensetzung nach einem der Ansprüche 1 bis 10 zum Gebrauch bei einer Behandlung von Gingiva- oder Zahnfleischrückgang, umfassend ein Verhindern von Gingivarückgang, ein Stoppen von Gingivarückgang, ein Rückgängigmachen von Gingivarückgang, ein Verringern von Zahnfleischrückgang, ein Fördern einer Kollagensynthese, das Fördern einer Synthese einer extrazellulären Matrix, ein Steigern einer Zahnfleischelastizität, das Steigern einer Epidermisdicke, das Fördern einer Fibrillinsynthese oder Kombinationen davon in einer Mundhöhle eines Benutzers oder eines Tieres, umfassend ein Inkontaktbringen der Mundpflegezusammensetzung nach einem der Ansprüche 1 bis 10 mit wenigstens einer Oberfläche der Mundhöhle.

12. Kit, umfassend:
(a) die zum Belassen in dem Mund bestimmte Mundplfegezusammensetzung nach einem der Ansprüche 1 bis 10; und
(b) einen Abgabeträger.

13. Kit nach Anspruch 12, wobei der Abgabeträger Streifen, Materialfolie, Zahnschiene, Aligner, Schwammmaterial, Applikator oder Mischungen davon umfasst.

## Revendications

1. Composition de soins bucco-dentaires comprenant :
(a) composé de santé gingivale, de préférence dans lequel le composé de santé gingivale comprend une source d'ions métalliques, une vitamine, de l'allantoïne, un composé de renforcement de la gomme, ou des combinaisons de ceux-ci ; et
(b) acide aminé, dans lequel l'acide aminé comprend un ou plusieurs acides aminés, peptides et/ou polypeptides et
dans lequel le composé de santé gingival comprend le rétinol et l'acide aminé comprend le pal-KTTKS,
de préférence, la composition de soins bucco-dentaires est exempte d'abrasif, ou plus préférablement, le composé de santé gingivale est exempt de composé d'acide jasmonique, d'acide gibbérellique, de composé de zéatine, ou de combinaisons de ceux-ci.

2. Composition de soins bucco-dentaires de la revendication 1, dans laquelle la source d'ions métalliques comprend de l'étain, du zinc, du cuivre ou des combinaisons de ceux-ci, de préférence dans laquelle l'étain comprend du fluorure stanneux, du chlorure stanneux ou des combinaisons de ceux-ci.

3. La composition de soins bucco-dentaires de la revendication 1 ou 2, dans laquelle la vitamine comprend la vitamine A, la vitamine B, la vitamine C, la vitamine D, la vitamine E, des dérivés de celles-ci ou des combinaisons de celles-ci, de préférence dans laquelle la vitamine comprend un composé rétinoïde, plus préférentiellement dans laquelle le composé rétinoïde comprend la vitamine A, un vitaminé de la vitamine A, un dérivé de la vitamine A, ou des combinaisons de ceux-ci, ou encore plus préférablement dans lequel le composé rétinoïde comprend l'ester de rétinyle, le rétinal, l'acide rétinoïque, le tocopheryl-retinoate, l'ester de tocophérol de l'acide cis- ou *trans-rétinoïque,* l'isotrétinoïne, l'alitrétinoïne, l'etretinate, l'acitrétine, l'adapalène, le bexarotène, le tazarotène, ou des combinaisons de ceux-ci.

4. Composition de soins bucco-dentaires de la revendication 3, dans laquelle l'ester de rétinyle comprend le palmitate de rétinyle, l'acétate de rétinyle, le propionate de rétinyle ou des combinaisons de ceux-ci.

5. La composition de soins bucco-dentaires de l'une des revendications 1 à 4, dans laquelle l'acide aminé comprend un acide aminé basique, un acide aminé neutre, un acide aminé acide, ou des combinaisons de ceux-ci, de préférence dans laquelle l'acide aminé comprend un acide aminé naturel, un acide aminé synthétique, et des combinaisons de ceux-ci, ou plus préférentiellement dans laquelle l'acide aminé comprend de l'arginine, histidine, lysine, acide aspartique, acide glutamique, sérine, thréonine, asparagine, glutamine, cystéine, sélénocystéine, glycine, proline, alanine, valine, isoleucine, leucine, méthionine, phénylalanine, tyrosine, tryptophane, citrulline, ornithine, créatine, acide diaminobutonique, acide diaminoproprionique, leurs sels ou leurs combinaisons.

6. La composition de soins bucco-dentaires de l'une des revendications 1 à 5, dans laquelle l'acide aminé comprend un peptide, un polypeptide ou des combinaisons de ceux-ci, de préférence dans laquelle le peptide comprend de deux acides aminés à dix acides aminés, ou plus préférentiellement dans laquelle le peptide comprend un dipeptide, un tripeptide, un tétrapeptide, un pentapeptide ou des combinaisons de ceux-ci.

7. La composition de soins bucco-dentaires de la revendication 6, dans laquelle le peptide comprend la lysine, la thréonine, la sérine, la glycine, l'acide glutamique, l'histidine, la proline, ou des combinaisons de ceux-ci, de préférence dans laquelle le peptide comprend un peptide avec une séquence de glycine-histidine-lysine, glycine-acide glutamique-lysine-glycine.

8. La composition de soins bucco-dentaires de l'une des revendications 1 à 7, dans laquelle la composition a un coefficient de consistance de la viscosité K de 20 Pa-s à 500 Pa-s, mesuré à 22 °C à un taux de cisaillement de 0,^{1-10s-1}, de préférence dans laquelle la composition de soins bucco-dentaires a un indice de mucoadhésion d'au moins 0.3 Pourcentage d'intensité de fluorescence ("FI%"), de préférence dans laquelle la composition comprend un polymère mucoadhésif, ou de préférence encore dans laquelle le polymère mucoadhésif comprend de l'acide polyacrylique, de la gomme naturelle, un polysaccharide sulfaté linéaire, de la cellulose anionique, un dérivé de cellulose non ionique, de la polyvinylpyrrolidine, de l'acide hyaluronique, ou des combinaisons de ces éléments.

9. La composition pour soins bucco-dentaires de l'une des revendications 1 à 8, dans laquelle la composition comprend du fluorure, de préférence du fluorure stanneux, du fluorure de sodium, du monofluorophosphate de sodium, du fluorure d'amines, ou des combinaisons de ceux-ci.

10. Composition de soins bucco-dentaires de l'une des revendications 1 à 9, dans laquelle le composé de santé gingivale comprend du rétinol de qualité pharmaceutique.

11. La composition de soins bucco-dentaires de l'une des revendications 1 à 10 pour une utilisation dans le traitement de la récession gingivale ou gingivale comprenant la prévention de la récession gingivale, l'arrêt de la récession gingivale, l'inversion de la récession gingivale, la diminution de la récession gingivale, la promotion de la synthèse du collagène, la promotion de la synthèse de la matrice extracellulaire, l'augmentation de la résilience gingivale, l'augmentation de l'épaisseur de l'épiderme, la promotion de la synthèse de la fibrilline, ou des combinaisons de ceux-ci dans la cavité buccale d'un utilisateur ou d'un animal comprenant le contact de la composition de soins bucco-dentaires de l'une des revendications 1 à 10 avec au moins une surface de la cavité buccale.

12. Trousse, comprenant :
(a) la composition selon l'une quelconque des revendications 1 à 10 ; et
(b) un véhicule d'administration,

13. Le kit de la revendication 12, dans lequel le support d'administration comprend une bande, un film de matériau, une gouttière dentaire, un aligneur, un matériau éponge, un applicateur, ou des mélanges de ceux-ci.
